# EUROPÄISCHE PATENTSCHRIFT

(11) **EP 3 350 340 B1**
(45) Veröffentlichungstag und Bekanntmachung des Hinweises auf die Patenterteilung: **14.04.2021**
(21) Anmeldenummer: 16767225.2
(22) Anmeldetag: 15.09.2016
(51) Int. Cl.: C12Q 1/68

(54) **BESTÄTIGUNGSTEST FÜR PRIMÄRE NUKLEINSÄURE-AMPLIFIKATE IN EINEM KONTINUIERLICHEN REAKTIONSANSATZ UND UNMITTELBARE AUSWERTUNG MITTELS IMMUNCHROMATOGRAPHISCHER VERFAHREN**
CONFIRMATION TEST FOR PRIMARY NUCLEIC ACID AMPLIFICATION PRODUCTS IN A CONTINUOUS REACTION BATCH AND IMMEDIATE EVALUATION BY MEANS OF IMMUNOCHROMATOGRAPHIC METHODS
TEST DE VÉRIFICATION DE PRODUITS D'AMPLIFICATION PRIMAIRES D'ACIDES NUCLÉIQUES DANS UN PROCESSUS RÉACTIONNEL CONTINU ET ANALYSE DIRECTE AU MOYEN D'UN PROCÉDÉ IMMUNOCHROMATOGRAPHIQUE

(30) Priorität: 18.09.2015 DE 102015115836
(43) Veröffentlichungstag der Anmeldung: 25.07.2018
(73) Patentinhaber: Biotype GmbH, 01109 Dresden (DE)
(72) Erfinder: BRABETZ, Werner, 01097 Dresden (DE)
(74) Vertreter: Heide, Anna Katharina
(86) Internationale Anmeldenummer: PCT/EP2016/071746
(87) Internationale Veröffentlichungsnummer: WO 2017/046191

(56) Entgegenhaltungen:
- EP-A1- 2 208 795
- WO-A1-2009/155271
- WO-A1-2012/067831
- WO-A1-2012/096430
- WO-A1-2015/024948
- US-A1- 2014 087 382
- Mehlig et al.: "DNA-probe based detection for differential diagnosis of dermatomycoses", Researchgate, 1. September 2014 (2014-09-01), XP002765191, Gefunden im Internet: URL:https://www.researchgate.net/publicati on/291426099_DNA-probe_based_detection_for _differential_diagnosis_of_dermatomycoses [gefunden am 2016-12-13]
- HATAIRAT KAMPHEE ET AL: "Rapid Molecular Detection of Multidrug-Resistant Tuberculosis by PCR-Nucleic Acid Lateral Flow Immunoassay", PLOS ONE, Bd. 10, Nr. 9, E0137791, 10. September 2015 (2015-09-10), Seiten 1-17, XP055328706, DOI: 10.1371/journal.pone.0137791

## Beschreibung

Die vorliegende Erfindung betrifft ein Verfahren zur Bestätigung von amplifizierten Nukleinsäure-Zielsequenz (Zielsequenz), vorzugsweise aus humanen Proben, während einer Vervielfältigungsreaktion in einem kollektiven und kontinuierlichen Reaktionsansatz als Eintopfverfahren, worin die Bestätigung des Zielsequenzamplifikats über ein Haptenpaarmarkiertes artifizielles Matrizenamplifikat erhalten wird. Dabei wird die artifizielle Matrizensequenz mittels der 5'-Spaltprodukte der mindestens einen Zielsequenz-spezifischen FEN-Sonde amplifiziert und optional markiert. Das 5'-Spaltprodukt der mindestens einen Zielsequenz-spezifischen FEN-Sonde wird nur dann erhalten, wenn die FEN-Sonde mit ihrer Zielsequenz-spezifischen 3'-Sequenz an einen komplementären Sequenzabschnitt der mindestens einen Zielsequenz hybridisiert. Die Detektion der erhaltenen Vielzahl an Matrizenamplifikaten erfolgt distinkt und bevorzugt mittels immunchromatographischer Verfahren.

Nukleinsäureamplifikationstechnologien (NAT), allen voran die Polymerase-Kettenreaktion (PCR), sind heute wesentlicher Bestandteil der molekulargenetischen Diagnostik. Dabei sind auch mehrere Parameter gleichzeitig in so genannten Multiplex-Verfahren nachweisbar.

Zur Erhöhung der Spezifität und insbesondere zur Qualitätssicherung des molekularen Nukleinsäurenachweises von Krankheitserregern wird ein Bestätigungstest für die Amplifikationsprodukte gefordert (MIQ-1 2011, Rili-BÄK-B3 2013). Hierfür werden beispielsweise in homogenen Testverfahren (so genannte Eintopfverfahren oder -reaktionen wie z. B. die Echtzeit-PCR) vielfach DNA-Sonden verwendet, welche mit einem DNA-Halbstrang des DNA-Amplifikats sequenzspezifisch hybridisieren und mit einem Fluorophorpaar, welches über FRET (Förster Resonanz Energie Transfer) wechselwirkt, markiert sind. Der FRET wird durch die sequenzspezifische Hybridisierung moduliert oder aufgehoben, sodass ein mittels Echtzeit-Thermocycler messbares Signal entsteht. Ein bevorzugtes Sondenformat sind so genannte Hydrolysesonden, welche nach der Hybridisierung durch die intrinsische Nukleaseaktivität der *Taq* DNA Polymerase gespalten werden, wodurch das Fluorophorpaar getrennt wird.

Alternativ zu homogenen Tests können als Bestätigungstest der NAT nachgeschaltete Verfahrensschritte wie Hybridisierung der DNA-Amplifikate an immobilisierten Sonden (z. B. Reverse-Line-Blot, Lateralflow Immunchromatographie Test, DNA-Chip, DNA-Bead-Assay), Southern-Hybridisierung, DNA-Sequenzierung oder Nested-PCR durchgeführt werden. Diese Strategien erfordern jedoch zusätzliche Prozessierungsschritte für den Bestätigungstest, welche in Bezug auf Zeit und Kosten aufwendiger sind und die Gefahr einer Kontamination beinhalten.

Eine Kontamination der Amplifikate erfolgt beim Entnehmen eines Aliquots aus dem PCR-Reaktionsansatz und/oder beim Überführen dieses Aliquots in einen neuen Reaktionsansatz für das nachgeschaltete Verfahren zur Bestätigung der Amplifikate. Eine Kontamination umfasst insbesondere die Verunreinigung des Amplifikats mit Fremd-DNA oder Amplifikaten aus parallel prozessierten Proben (Kreuzkontamination).

Eine Kontamination hat enorme Auswirkungen auf die Qualität des Bestätigungstests. Unter Berücksichtigung des hochsensiblen Anwendungsbereichs solcher Bestätigungstests, wie Diagnostik, Tumordiagnostik, Diagnostik von schwerwiegenden Infektionserregern und deren Resistenzen, entscheidet ein Bestätigungstest über die Diagnose und die daraus resultierende Therapie eines Patienten. Fehldiagnosen führen zu falschen Therapien, Folgeschäden beim Patienten sowie erhöhte Kosten für das Gesundheitssystem.

Daher ist ein verlässlicher Bestätigungstest wesentlich. Dieser sollte einfach und robust sein, sodass Fehlerraten möglichst reduziert werden.

Die Bestätigung von NAT-Amplifikaten mittels Fluoreszenzfarbstoffen, welche doppelsträngige DNA sequenzunspezifisch binden (z. B. SYBR Green I), oder ausschließlich über die Größenbestimmung der primären Amplifikate durch nachgeschaltete DNA-Elektrophoresen (Flachgel- oder Kapillargelelektrophorese) sind keine anerkannte Verfahren gemäß der genannten Richtlinien.

Ein Nachteil homogener Testverfahren ist die relativ geringe Multiplexfähigkeit der Echtzeit-Thermocycler, welche zurzeit nur über 3-6 verschiedene Detektionskanäle verfügen.

Darüber hinaus besteht die große Notwendigkeit für molekulargenetische Testformate, um patientennahe (Point-of-Care) oder dezentrale Diagnostik am Ort des Bedarfs (Point-of-Need) zu erschließen. Dies gilt insbesondere für die Eindämmung von Krankheitserregern und den Nachweis derer Antibiotikaresistenzen in ressourcenarmen Umgebungen. Lateralflow Immunchromatographietests (LFT) haben sich in diesen Anwendungsgebieten für den Nachweis von Antigenen und anderen Bindungsliganden bereits bewährt (Hu *et al.* 2014).

LFT sind insbesondere für den Nachweis von Analyten aus den Stoffgruppen der Proteine, Kohlenhydrate sowie bestimmte Drogen und Toxine gut etabliert. Für die Praktikabilität von LFT, welche für den Nachweis von Nukleinsäureamplifikaten gedacht sind (Nucleic Acid Lateral Flow Test, NALFT), bestehen jedoch vielfach noch technische Einschränkungen. Dies betrifft, wie im Folgenden ausgeführt, insbesondere auch einen Bestätigungstest für Nukleinsäure-Amplifikationsprodukte.

Die WO2012/096430A1 offenbart den Nachweis einer Zielnukleinsäuresequenz durch einen PTOCE-Assay (PTO Cleavage and Extension). Dabei wird eine Zielnukleinsäuresequenz detektiert, in der das mit der Zielnukleinsäuresequenz hybridisierte PTO (Probing and Tagging Oligonukleotid) gespalten wird, um ein 5'-Fragment freizusetzen. Das 5'-Fragment wird mit dem CTO (Capturing and Templating Oligonukleotid) hybridisiert, um ein markiertes Duplex zu bilden, das dann wiederum in einer Schmelzkurve nachgewiesen wird. Die Verwendung der 5'-Fragmente als Primer zur Amplifikation einer Nukleinsäure wird nicht offenbart.

DE10154291B4 beschreibt z. B. einen Schnelltest, bei welchem nach einer PCR oder Multiplex-PCR eine DNA-Hybridisierung mit immobilisierten zielsequenzspezifischen Oligonukleotidsonden im Detektionsbereich eines LFT-Streifens erfolgt. Des Weiteren offenbart EP1623042B1 eine DNA-Hybridisierung mit einzelsträngigen Sonden, welche in der Konjugatzone eines LFT-Streifens vorliegen. Beide Verfahren erfordern denaturierende Laufpuffer und sequenzspezifische Anpassungen der Hybridisierungsbedingungen, welche insbesondere die Testentwicklung erschweren und kontrollierte Umgebungstemperaturen für die Durchführung des LFT voraussetzen.

Um die vorgenannten Anpassungen zu vermeiden, können im PCR-Ansatz die Hybridisierungssonden mitgeführt werden. Allerdings ist ein Nachteil dieser Strategie, dass die Hybridisierung der Sonde in Konkurrenz zur Primerverlängerung durch die DNA-Polymerase bzw. zur Rehybridisierung der DNA-Halbstränge der Nukleinsäureamplifikate steht, welche am Ende der NAT in hohen Konzentrationen vorliegen. Bei Verwendung der weit verbreiteten *Taq* DNA Polymerase müssen zusätzliche Signalverluste durch die intrinsische Nukleaseaktivität des Enzyms berücksichtigt werden. Aufgrund dieser Abhängigkeiten ergibt sich ein Verlust in der Qualität des Verfahrens. Dadurch wurde im Rahmen der Versuche der vorliegenden Erfindungen an einem Lösungsansatz zur Verhinderung der vorgenannten Nachteile gearbeitet, um die Einschränkungen der vorgenannten Tests aus dem Stand der Technik zu beheben.

Im Gegensatz können Hapten-basierte LFT-Streifen mit einem universellen Laufpuffer bei variablen Umgebungstemperaturen eingesetzt werden. Diese sind in der Detektionszone mit Hapten-spezifischen Rezeptormolekülen (z. B. Antikörper, Streptavidin) funktionalisiert. Zum visuellen oder Geräte gestützten optischen Nachweis wird im Konjugatreservoir des LFT-Streifens beispielsweise kolloidales Gold vorgelegt, welches mit einem zweiten unabhängigen Hapten-spezifischen Rezeptormolekül konjugiert ist. Verfahren zur Markierung von Oligonukleotiden mit verschiedenen Haptenen (z. B. Biotin, Digoxigenin, o-Nitrophenol, Peptide, Fluorophore) sind beschrieben. Ein DNA-Amplifikatnachweis gelingt, indem während der PCR nur ein DNA-Halbstrang über einen Primer markiert wird, welcher z. B. das Hapten des kolloidalen Goldkonjugates trägt. Ein Aliquot des PCR-Amplifikats wird anschließend im Laufpuffer verdünnt und in Gegenwart einer targetsequenzspezifischen Oligonukleotidsonde inkubiert, welche mit dem bereits einfach markierten DNA-Halbstrang hybridisiert und mit einem Hapten markiert ist, welches an ein Rezeptormolekül der Detektionszone bindet. Dieser zusätzliche, der PCR nachgeschaltete Hybridisierungsschritt zeigt jedoch die oben genannten Nachteile und sollte deshalb vermieden werden.

Daher war es Ziel der vorliegenden Erfindung die Bereitstellung von Bestätigungstests für diagnostische Tests, die an die Durchführung entsprechender Bestätigungstests gebunden sind, zu erschließen und/oder zu vereinfachen. Es wird ein vereinfachter Bestätigungstests (synonym Bestätigungsverfahren) für NAT für einen NALFT für patientennahe (Point-of-Care) oder dezentrale Diagnostik am Ort des Bedarfs (Point-of-Need) vorgestellt.

Aufgabe der vorliegenden Erfindung ist es einen kontinuierlichen und kollektiven Reaktionsansatz für die Amplifikation von Zielsequenzen, insbesondere aus humanen Proben wie Blut, Plasma, Knochen und/oder Gewebe, und gleichzeitig für den darauf folgenden Bestätigungstest bereitzustellen. Es ist ebenfalls das Ziel ein Verfahren, insbesondere ein Eintopfverfahren, zur Amplifikation und Bestätigung der jeweiligen Zielsequenz unter Verwendung des vorgenannten Reaktionsansatzes bereitzustellen. Dabei sollen Zwischenschritte zur weiteren Prozessierung der erhaltenen Amplifikate, wie Aufreinigung und/oder zusätzliche Sondenhybridisierungen in getrennten Gefäßen vermieden werden. Somit ist ein weiteres Ziel der vorliegenden Erfindung das Risiko der Kontamination mit Fremd-DNA, RNA, Proteine, Peptide und/oder Chemikalien zu vermeiden und ferner ein vereinfachtes und schnelleres Verfahren bereitzustellen. Es soll ein Verfahren bereitgestellt werden, dass die vorgenannten Nachteile und Risiken aus dem Stand der Technik vermeidet und gleichzeitig die Vorteile und Potentiale bestehender Multiplex-Nachweisverfahren kombiniert. Daher ist eine weitere Aufgabe die Bereitstellung eines Verfahrens zur Amplifikation mindestens einer Zielsequenz, insbesondere aus einer humanen Probe eines Patienten, und eine kontinuierlich folgende Bestätigung der mindestens einen Zielsequenz und Detektion mittels eines immunchromatographischen Verfahren, wie des Nukleinsäure-Lateral-Flow (NALF)-Verfahren. Somit ist ein weiteres Ziel der vorliegenden Erfindung die Bereitstellung eines Verfahrens, das mit einem kontinuierlichen Reaktionsansatz und dem daraus erhaltenen Amplifikaten variablen Detektionsverfahren mit einer festen Phase ohne Prozessierungsschritte zugeführt werden kann. Es soll ein Verfahren für die Diagnostik, insbesondere Humandiagnostik, zur Verfügung gestellt werden, das eine geringere Fehlerrate bei der Bestätigung des jeweiligen Amplifikats aufweist, da das Risiko Kontamination verringert bis zu verhindert wird. Es soll auch ein Bestätigungsverfahren mit hoher Sensitivität für Proben mit geringer Qualität und/oder sehr geringer verwertbarer DNA-Menge bereitgestellt werden. Der Reaktionsansatz, das Multiplex-Kit und das Bestätigungsverfahren sollen einfach und ortsunabhängig (Point-of-Need) bei gleich hoher Qualität verwendet und durchgeführt werden können. Ein wesentliches Ziel ist es einen Bestätigungstest bereitzustellen, der die Anforderungen mindestens der Richtlinie MIQ-1 2011 für Nukleinsäuren-Amplifikations-Techniken und/oder die Richtlinie der Bundesärztekammer B3 (Rili BÄK-B3) für den direkten Nachweis und Charakterisierung von Infektionserreger erfüllt sowie auch jeweils die Anforderungen der jeweiligen Novellierungen der Richtlinie erfüllt.

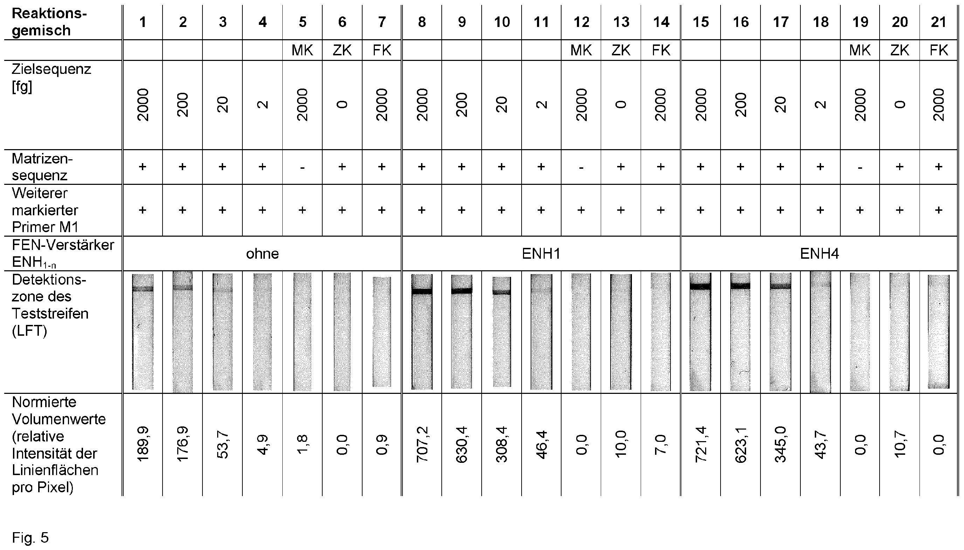

Daher stellt die vorliegende Erfindung ein Verfahren bereit, das durch den erfindungsgemäßen Bestätigungstest (synonym = Bestätigungsverfahren) gekennzeichnet ist, in dem eine homoge PCR oder Multiplex-PCR ohne zusätzliche Pipettierschritte (Eintopfverfahren) durchgeführt wird. Die wesentlichen Vorteile der vorliegenden Erfindung sind, dass ein Eintopfverfahren zur Bestätigung von Zielsequenzen bereitgestellt wird und das erfindungsgemäße homogene Testformat die Möglichkeit zur Quantifizierung der Ausgangsnukleinsäuren bereitstellt, wie nachfolgend beschrieben. Dadurch ist ein weiterer Vorteil der vorliegenden Erfindung, dass Proben mit nur sehr geringem Anteil an verwertbaren DNA-Ausgangsmaterialien und/oder degradierte DNA-Ausgangsmaterialien dennoch als Zielsequenz verwendet und erfindungsgemäß bestätigt und detektiert werden können. Für ausgewählte Kombinationen (Tabelle 3) der Komponenten des erfindungsgemäßen Reaktionsgemisches wird in Beispiel 3, Fig. 5, ein erfolgreicher Nachweis von sehr geringen DNA-Mengen gezeigt.

Die bevorzugt verwendeten Sonden sind so genannte Hydrolysesonden, welche direkt im PCR Reaktionsgemisch enthalten sind und nach der Hybridisierung an die Zielsequenz durch die intrinsische Nukleaseaktivität der *Taq* DNA Polymerase gespalten werden. Dadurch können z. B. in der HIV-Diagnostik die Viruslast des Patienten sowie nach Medikation der Therapieerfolg ermittelt werden. Das erfindungsgemäße Bestätigungsverfahren hat den besonderen Vorteil, dass erfindungsgemäße FEN-Sonden, welche am Ende der Vervielfältigungsreaktion, insbesondere PCR ggf. noch im Überschuss vorliegen, nicht reaktiv sind. Damit behindern diese überschüssigen FEN-Sonden die Bestätigung und Detektion der erhaltenen Matrizenamplifikate nicht.

Das erfindungsgemäße Bestätigungsverfahren hat somit den Vorteil durch die exponentielle Signalverstärkung sensitiver zu sein und erlaubt einen höheren Multiplex-Grad.

Des Weiteren kann der Bestätigungstest als standardisierte universelle Reaktionsschritte unabhängig von der zu amplifizierenden Zielnukleinsäure konzipiert werden. Letzteres erlaubt die Ausarbeitung eines testunabhängigen Entwicklungswerkzeugs bestehend aus universellen PCRs und/oder universellen Primerverlängerungsreaktion.

Die Reaktionsprodukte (synonym = Amplifikate, Matrizenamplifikate) des Bestätigungstests sind kompatibel mit den Standardverfahrensschritten zum Nachweis mittels Lateralflow Immunchromatographie.

Die erfindungsgemäße Lösung wird im Folgenden beschrieben. Ausgewählte Ausführungsbeispiele zeigen Wege zur Erzielung der erfindungsgemäßen Lösung und erläutern das Grundprinzip und Funktionsweise der vorliegenden Erfindung, wobei die hier präsentierten Beispiele nicht beschränkend auszulegen sind.

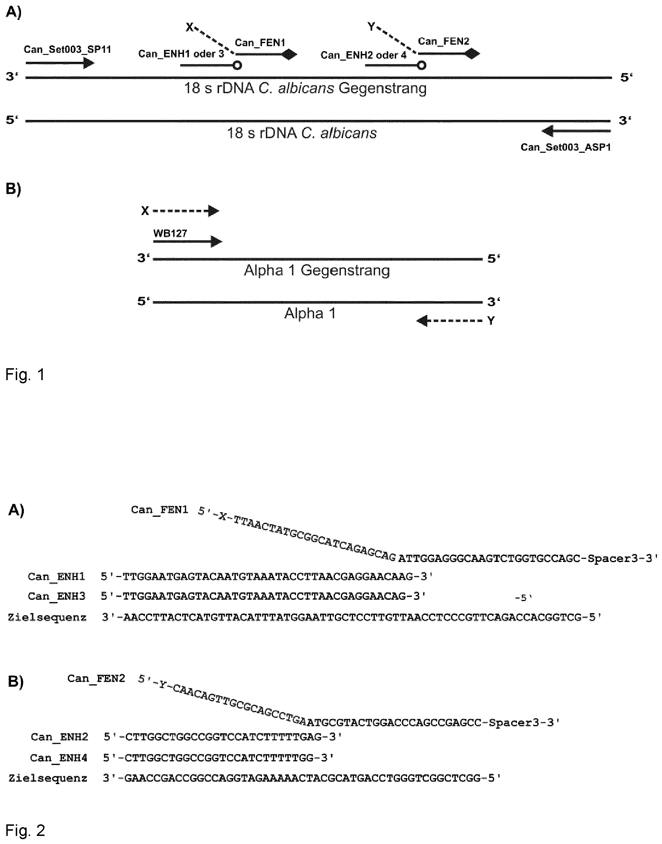

Ein erster Gegenstand der vorliegenden Erfindung ist eine Bestätigung, insbesondere ein Bestätigungsverfahren, mindestens einer amplifizierten Nukleinsäure-Zielsequenz, insbesondere DNA und/oder cDNA, welche nachfolgend kurz als Zielsequenz bezeichnet wird, während einer Vervielfältigungsreaktion in einem kollektiven und kontinuierlichen Reaktionsansatz enthaltend ein Reaktionsgemisch umfassend
- mindestens eine nachzuweisende Zielsequenz,
- mindestens zwei Zielsequenz-spezifische Primer (P1, P2, P₁₋ₙ), die zur Amplifikation der mindestens einen Zielsequenz geeignet sind,
- mindestens eine markierte Zielsequenz-spezifische, insbesondere intermolekulare, Flap-Endonukleasesonde (FEN-Sonde FEN1, FEN₁₋ₙ), wobei die mindestens eine, insbesondere einzelsträngige, FEN-Sonde umfasst
   - eine Zielsequenz-spezifische 3'-Sequenz, insbesondere DNA 3'-Sequenz, welche komplementär zu einem Sequenzabschnitt der mindestens einen Zielsequenz innerhalb einer Region ist, die von dem mindesten ersten Primer (P1) und dem mindestens zweiten Primer (P2) auf der Zielsequenz begrenzt wird, insbesondere hybridisieren P1 und P2 mit der Zielsequenz (siehe Fig. 1)
   - am 3'-Ende der Zielsequenz-spezifischen 3'-Sequenz eine Schutzgruppe, insbesondere als Polymeraseblocker, vorzugsweise fehlt die 3'-OH-Gruppe oder sie ist kovalent modifiziert, und
   - eine Zielsequenz-unspezifischen 5'-Sequenz, insbesondere DNA 5'-Sequenz, welche an ihrem 5'-Ende mit einem Hapten eines spezifischen Haptenpaares markiert ist, insbesondere mit einem Detektor-Hapten oder einem zur Matrizensequenz sequenzspezifisches Hapten markiert ist und
- mindestens eine artifizielle Matrizensequenz, insbesondere artifizielle Nukleinsäuresequenz,
- mindestens einen weiteren Primer (M1), wobei der weitere Primer (M1) komplementär zu einem Sequenzabschnitt des Gegenstrang der mindestens einen artifizielle Matrizensequenz ist und/oder
- mindestens ein FEN-Verstärker-Oligonukleotid (ENH1-n)
   das Bestätigungsverfahren umfasst je Zyklus der Vervielfältigungsreaktion die Schritte
- Hybridisierung der Zielsequenz-spezifischen 3'-Sequenz der mindestens einen markierten FEN-Sonde an eine komplementäre Sequenz der mindestens einen nachzuweisenden Zielsequenz,
- Spaltung der mindestens einen markierten FEN-Sonde (FEN₁₋ₙ),
- insbesondere Erhalt von mindestens einem freien am 5'-Ende mit einem Hapten eines spezifischen Haptenpaares markierten 5'-Spaltprodukt (S₁₋ₙ) umfassend jeweils die Zielsequenz-unspezifische 5'-Sequenz,
- Hybridisierung des mindestens einen markierten 5'-Spaltprodukts (S₁₋ₙ) der mindestens einen FEN-Sonde (FEN₁₋ₙ) an eine komplementäre Sequenz der mindestens einen artifiziellen Matrizensequenz, insbesondere an die Matrizensequenz eines denaturierten Doppelstrangs, insbesondere eines Einzelstrangs,
- insbesondere Elongation des an der vorzugsweise einzelsträngigen artifiziellen Matrizensequenz mindestens einen hybridisierten markierten 5'-Spaltprodukts (S₁₋ₙ)
- Amplifikation der mindestens einen artifiziellen Matrizensequenz mit dem mindestens einen markierten 5'-Spaltprodukt (S₁₋ₙ) der mindestens einen FEN-Sonde und dem mindestens einen Primer (M1), und
- Markierung der mindestens einen artifiziellen Matrizensequenz während der Amplifikation durch das mindestens eine 5'-Spaltprodukt (S₁₋ₙ) der mindestens einen FEN-Sonde (FEN1, FEN₁₋ₙ), vorzugsweise durch das eine Hapten eines spezifischen Haptenpaares vom 5'-Ende des 5'-Spaltprodukts (S1, S₁₋ₙ) und durch ein zweites Hapten eines 5'-Spaltprodukt (S2, S₁₋ₙ) einer zweiten FEN-Sonde (FEN2, FEN₁₋ₙ) oder eines weiteren Primers (M1), und
- insbesondere Erhalt mindestens eines, vorzugsweise markierten artifiziellen Matrizensequenzamplifikats (synonym = artifizielles Matrizenamplifikat, Matrizenamplifikat) und
- insbesondere Detektion des mindestens einen optional markierten Matrizensequenzamplifikats, vorzugsweise wird das optional markierte Matrizensequenzamplifikat in einer Flüssigphase detektiert, besonders bevorzugt mittels eines immunchromatographischen Verfahrens.

Das vorbeschriebene Verfahren mit dem kontinuierlichen und kollektiven Reaktionsansatz für die Amplifikation der Zielsequenz und Bestätigung der erhaltenen Zielsequenzamplifikate mittels einer Amplifikation und Markierung einer Matrizensequenz kann synonym als Eintopfverfahren bezeichnet werden, da die vorgenannten Reaktionen ohne Prozessierungsschritte und ohne zeitliche oder räumliche Trennung erfolgen. Der Fachmann versteht, dass alle weiteren Komponenten, wie z. B. Puffersystem, Nukleotide, Salze, etc., die für eine erfolgreiche PCR erforderlich sind, ebenfalls im Reaktionsgemisch enthalten sind. Nach Erhalt des optional markierten Matrizensequenzamplifikats kann die Detektion mit dem gewünschten Verfahren und Gerät jederzeit und ortsunabhängig durchgeführt werden.

Eine Zielsequenz ist eine Nukleinsäure-Sequenz innerhalb einer Probe (synonym = Untersuchungsgut), die im Rahmen einer Analytik oder Diagnostik z. B. dem spezifischen Nachweis eines Individuums (Forensik, Genealogie), einer Art (z. B. Krankheitserreger, genetisch veränderter Organismus), einer Krankheit oder eines anderen biologischen Merkmals dient. Die Probe umfasst alle denkbaren Ausgangsmaterialien mit biologischem Anteil wie z. B. pflanzliche, tierische und menschliche Flüssigkeiten, extrazelluläre Kreislaufflüssigkeiten, insbesondere Blut, Plasma, Serum und/oder Lymphe, Verdauungssäfte, insbesondere Speichel, Magensaft, Saft der Pankreas und/oder Galle, Sekrete und Exkrete, insbesondere Schweiß, Urin, Kot, Ejakulat, Vaginalsekrete, Tränenflüssigkeit, Nasensekret und/oder Muttermilch und/oder weitere Flüssigkeiten oder Absonderungen, insbesondere Fruchtwasser, Ohrenschmalz, Hirnwasser und/oder Eiter und Gewebe, Nägel, Haare und/oder Knochenbestandteile, Nahrungsmittel, Umweltisolate etc., und/oder synthetische Nukleinsäuren (z. B. Barcode-Sequenzen und andere gezielte DNA-Markierung von anderen Artikeln), und kann eine oder mehrere Zielsequenzen umfassen. Probe umfasst ebenfalls Biopsie- und Abstrichmaterial. Vorzugsweise ist die Probe eine humane Probe.

In dem vorgenannten Verfahren ist die Zielsequenz bevorzugt eine DNA, insbesondere natürliche DNA und/oder cDNA (english *complementary DNA,* deutsch *komplementäre DNS*), die mittels einer Reversen Transkriptase aus RNA, wie mRNA oder ncRNA, synthetisiert wurde. Insbesondere in der medizinischen Diagnostik werden aus Proben verwertbare Ribonukleinsäuren zu cDNA umgeschrieben, um diese anschließend der Analytik, insbesondere dem erfindungsgemäßen Verfahren, als Zielsequenz zuzuführen. Insbesondere ist die erfindungsgemäß nachzuweisende Zielsequenz eine Zielsequenz, welche in multiplen Kopien pro Zelle vorliegt, umfassend mitochondriale DNA (mtDNA), rDNA, SINE (short interspersed nuclear element, *Alu*-Familie) und/oder MIR (mammalian-wide interspersed repeats). Vorzugsweise ist es eine doppelsträngige DNA.

Dabei wird erfindungsgemäß die Zielsequenz in einer Vervielfältigungsreaktion vervielfältigt (synonym amplifiziert), vorzugsweise in einer Vervielfältigungsreaktion einer Polymerasekettenreaktion (PCR) oder einer isothermalen Nukleinsäureamplifikationstechnologie (iNAT). Die PCR ist dem Fachmann bekannt. Unter Nukleinsäureamplifikationstechnologien (NAT) werden enzymatische Verfahren zur *in-vitro* Vervielfältigung von Nukleinsäuren, insbesondere von erfindungsgemäßen Zielsequenzen, bezeichnet. Diese können thermische Zyklen erfordern (z. B. PCR) oder isothermal (iNAT) verlaufen. Das erfindungsgemäße Verfahren kann zur Bestätigung beider Varianten eingesetzt werden. Weitere Ausführungsformen der vorgenannten Verfahren iNAT sind LAMP (loopmediated isothermal amplification), HDA (helicase-dependent amplification), RPA (recombinase polymerase amplification), SIBA (Strand Invasion Based Amplification), RCA (rolling circle amplification).

Erfindungsgemäß sind Flap-Endonukleasesonden, kurz als FEN-Sonden bezeichnet, Moleküle umfassend eine, insbesondere einzelsträngige Nukleinsäuresequenz, welche mindestens zwei funktionelle Bereiche aufweist. Die beiden funktionellen Bereiche sind eine 5'-Sequenz, die nicht komplementär zu einem Sequenzabschnitt der mindestens Zielsequenz ist (kurz Zielsequenz-unspezifische 5'-Sequenz genannt) und eine 3'-Sequenz, die komplementär zu einem Sequenzabschnitt der mindestens Zielsequenz ist (kurz Zielsequenz-spezifische 3'-Sequenz genannt). Der Sequenzabschnitt liegt innerhalb einer Region, die von dem mindesten ersten Primer (P1) und dem mindestens zweiten Primer (P2) auf der Zielsequenz begrenzt wird. Diese FEN-Sonden hybridisieren mit der Zielsequenz unter Ausbildung eines durch eine FEN abspaltbaren 5'-Flaps, der durch die Zielsequenz-unspezifische 5'-Sequenz dargestellt wird. Der Begriff Flap bezeichnet gabelförmig ungepaarte Strukturen innerhalb oder am Ende (3' oder 5') einer DNA-Doppelhelix. Diese Strukturen erkennt die Flap Endonuklease (FEN) als Substrat, wie es in Fig. 1 und Fig. 2 dargestellt ist. Das 3'-OH-Ende der FEN-Sonde ist erfindungsgemäß durch einen so genannten Polymeraseblocker gegen die Verlängerung durch eine DNA Polymerase geschützt. Durch Einwirkung einer FEN (Flap Endonuklease) wird die FEN-Sonde gespalten, wobei ein freies 5'-Spaltprodukt (S₁₋ₙ) erhalten wird.

FEN1 steht kurz für eine FEN-Sonde und FEN1 und FEN2 steht kurz für zwei FEN-Sonden. Entsprechend steht S1 für ein 5'-Spaltprodukt und S1 und S2 stehen für zwei 5'-Spaltprodukte.

Die Bezeichnung FEN₁₋ₙ steht für mindestens eine bis variabel viele FEN-Sonden bzw. S₁₋ₙ für entsprechend viele 5'-Spaltprodukte der jeweiligen FEN-Sonde, wobei n gleich eine ganze Zahl ist. Insbesondere ist n eine ganze Zahl und vorzugsweise gleich 2, 3, 4, 5, 6, 7, 8, 9 oder 10 (FEN1-10 entspricht zehn, insbesondere verschiedene, FEN-Sonden) usw. bis kleiner gleich 50 FEN-Sonden. Die höchst mögliche Anzahl der FEN-Sonden in einem erfindungsgemäßen Reaktionsgemisch ist abhängig von dem zur Detektion verwendeten Verfahren. Die Anzahl der Detektionskanäle in dem verwendeten Gerät und die maximale distinkte Auflösung unterschiedlicher, insbesondere artifizieller und optional markierter Matrizenamplifikate limitiert die maximal verwendbare Anzahl der erfindungsgemäßen FEN-Sonden.

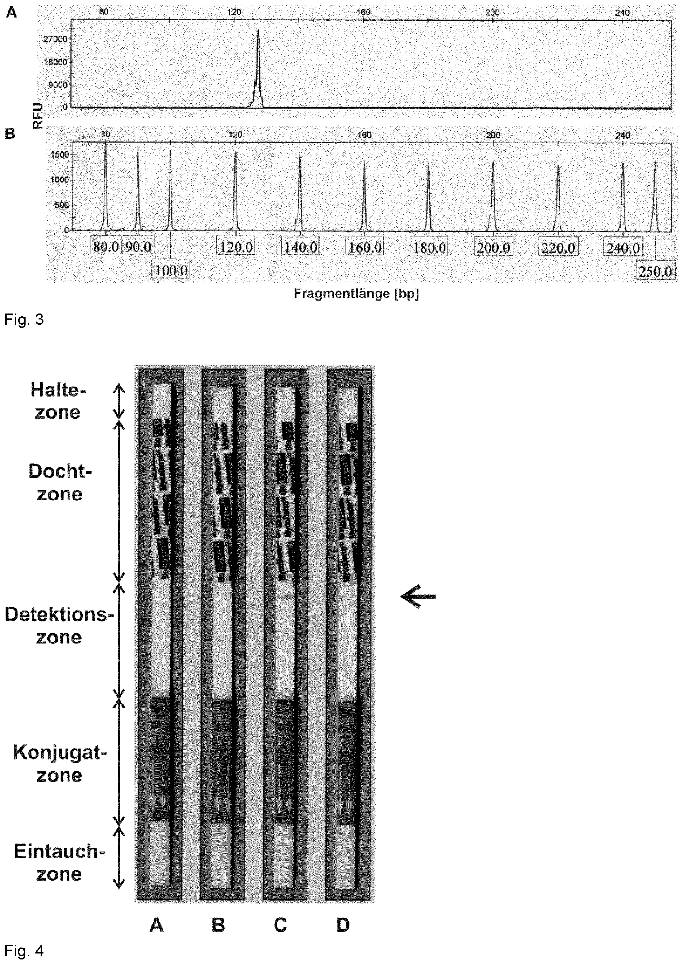

In Bezug auf das Detektionsverfahren basierend auf einer festen Phase zur Auftrennung der Matrizenamplifikate, insbesondere Lateralflow-Verfahren wie in Fig. 4 gezeigt, beeinflusst das Design der Festphase, insbesondere der Detektionszone, die maximal verwendbare Anzahl der erfindungsgemäßen markierten FEN-Sonden und/oder weiterer Primer (M1) sowie der artifiziellen Matrizensequenzen und damit die maximale mögliche Anzahl der distinkt nachweisbaren Matrizenamplifikate.

Die Schutzgruppe hat die Funktion eines Polymeraseblockers, wobei die Schutzgruppe das 3'-Ende eines Oligonukleotids gegen eine Verlängerung durch eine DNA Polymerase schützt. Dabei wird eine Erkennungsreaktion zwischen dem 3'-Ende der 3'-Sequenz der mindestens einen markierten FEN-Sonde und einer Polymerase verhindert, sodass das 3'-Ende nicht als Primer fungiert. Dies kann erfindungsgemäß erreicht werden durch das Fehlen der 3'-OH-Gruppe (3'-Didesoxynukleotid), durch chemische Modifikation der 3'-OH-Gruppe umfassend 3'-Phosphat, 3'-Spacer C3 (3'-Hydroxypropylphosphat), Amino, A-Alkyl, 3'-invertiertes Nukleotid, u. a. m. und/oder durch zusätzliche Nukleotide, welche nicht mit der Zielsequenz paaren.

Flap Endonukleasen (FEN) sind struktur- und strangspezifische Endonukleasen, welche die einzelsträngige DNA- oder RNA-Sequenz von einem gabelförmig ungepaarten 5'-Ende (5'-Flap) einer DNA-Doppelhelix abspalten (Lyamichev *et al.* 1993). FEN kommen bei allen Lebewesen vor und lösen in Verbindung mit weiteren Enzymen insbesondere während der DNA-Replikation die sogenannten Okazaki-Fragmente (RNA-DNA-Hybride) am zurückbleibenden Strang der Replikationsgabel auf (DNA-Reparaturfunktion). Eubakterielle FEN bilden zusammen mit einer DNA-Polymerase vom Typ Pol 1 (syonym = Pol A) eine Proteineinheit (z. B. Pol 1 von *Escherichia coli, Thermus aquaticus, T. thermophilus, Aquifex spp*.). Archaebakterielle (z. B. *Archaeoglobus fulgidus, Pyrococcus spp., Methanocaldococcus jannaschii, Methanothermobacter thermoautotrophicum*) und eukaryotische FEN (z. B. *Homo sapiens*) stellen eigenständige Proteine dar.

Die im Reaktionsgemisch enthaltende artifizielle Matrizensequenz (synonym auch Matrizensequenz oder Matrize genannt) ist eine Nukleinsäuresequenz, die bioinformatisch mit der minimalen Vorgabe entworfen wird, dass sie mit allen spezifischen Primer- und Sondenbindungsstellen der Zielsequenzen, welche im Multiplex verwendet werden, keine Übereinstimmung besitzt. Also weist die artifizielle Matrizensequenz keine komplementären Sequenzen zu den spezifischen Primer- und Sondenbindungsstellen der Zielsequenzen auf. Insbesondere darf sie für keinen Zielsequenz-spezifischen Primer des Multiplexes als DNA-Matrize fungieren. Die Enden der artifiziellen Matrizensequenz oder ihres Gegenstrangs tragen Bindungsstellen für unterschiedliche 5'-Spaltprodukte mindestens einer markierten FEN-Sonde, vorzugsweise zweier unterschiedlich markierter FEN-Sonden, oder für mindestens ein markiertes 5'-Spaltprodukt einer FEN-Sonde sowie eines zusätzlichen artifiziellen Primers M1. Das am 5'-Ende markierte 5'-Spaltprodukt einer FEN-Sonde besitzt ein freies 3'-OH-Ende und hat die Funktion eines Primers, welcher komplementär zur 5'→3'-Sequenz einer artifiziellen Matrizensequenz oder zum Gegenstrang der artifiziellen Matrizensequenz ist und ein wesentlicher Bestandteil des erfindungsgemäßen Bestätigungstest ist.

Unter einem weiteren Primer M1 (in den Beispielen als WB127 bezeichnet) wird ein Primer bezeichnet, welcher keine Kreuzhybridisierung mit allen Zielsequenzen des Multiplexes zeigt und mit dem Gegenstrang mindestens einer artifiziellen Matrizensequenz eine durch DNA Polymerase verlängerbare DNA-Doppelhelix ausbildet.

Multiplex beschreibt die Vervielfältigung und die Bestätigung mehrerer Zielsequenzen in einem Reaktionsansatz. Beispiele für Multiplex-Verfahren sind der genetische Fingerabdruck des Menschen durch Genotypisierung von 20 und mehr Short Tandem Repeats, die Differentialdiagnostik von verschiedenen somatischen Mutationen bei Tumoren, die Abklärung organspezifischer Infektionen (z. B. Lunge, Darm, sexuell übertragbare Infektionen) durch Nachweis spezifischer Erregergruppen und/oder die Amplifikation von Nukleinsäurebibliotheken (Panels). Das erfindungsgemäße Verfahren ist vorzugsweise ein Multiplex-Verfahren, welches für jede gewünschte Art - gleich oder analog zu den genannten Beispielen - eines Nachweises eingesetzt werden kann.

In einer Ausführungsform des erfindungsgemäßen Bestätigungsverfahrens umfasst die Schutzgruppe am 3'-Ende der 3'-Sequenz der mindestens einen, insbesondere einzelsträngigen, FEN-Sonde anstelle einer 3'-OH-Gruppe eine Nukleinsäuresequenz, insbesondere eine DNA-Sequenz größer gleich 1 Base bis kleiner gleich 5 Basen, welche zur Zielsequenz nicht komplementär ist. Vorzugsweise umfasst die Sequenz 1, 2, 3, 4 oder 5 Basen. Besonders bevorzugt sind 1 oder 2 Basen.

In einer weiteren Ausführungsform des erfindungsgemäßen Bestätigungsverfahren umfasst das Reaktionsgemisch ferner mindestens ein zur Spaltung der mindestens einen FEN-Sonde geeignetes Enzym, welches ausgewählt wird aus einer FEN als intrinsischer Bestandteil einer DNA Polymerase oder/oder als ein von einer Polymerase getrennt vorliegendes Enzym.

Bevorzugt umfasst das erfindungsgemäße Reaktionsgemisch mindestens eine Polymerase mit intrinsischer Endonukleaseaktivität, welche ausgewählt wird aus eubakteriellen FEN, die zusammen mit einer DNA Polymerase vom Typ Pol 1 (synonym = Pol A) eine Proteineinheit bilden. Daher hat diese Polymerase eine ihr innewohnenden, also intrinsische, FEN-Aktivität. Solche FEN sind in den Spezies z. B. *Escherichia coli, Thermus aquaticus, T. thermophilus* und/oder *Aquifex spp.* zu finden, dessen Polymerasen jeweils erfindungsgemäß eingesetzt werden können. Besonders bevorzugt wird im Sinne der Erfindung eine *Taq* DNA Polymerase mit intrinsischer FEN-Aktivität aus *Thermus aquaticus* eingesetzt.

Alternativ kann das Reaktionsgemisch erfindungsgemäß eine Polymerase und eine getrennt vorliegende FEN umfassen, wobei die FEN vorzugsweise ausgewählt wird aus archaebakteriellen FEN und/oder eukaryotischen FEN. In nachfolgenden Spezies *Archaeogiobus fulgidus, Pyrococcus spp., Methanocaldococcus jannaschii, Methanothermobacter thermoautotrophicum* und/oder *Homo sapiens* ist die FEN ein eigenständiges Protein, welches erfindungsgemäß eingesetzt werden kann. Vorzugsweise in Kombination mit einer Polymerase. Besonders bevorzugt werden thermostabile DNA Polymerasen und thermostabile FEN verwendet.

In einer weiteren Ausführungsform ist es denkbar, dass eine Polymerase mit intrinsischer FEN-Aktivität eingesetzt wird und zusätzlich eine getrennte FEN hinzugefügt wird. Dies ist dann vorteilhaft, wenn die Polymerase eine hervorragende Aktivität aufweist, aber ihre FEN-Aktivität nicht verlässlich ist, zu gering ist und/oder sonstige ungünstige biochemische Merkmale (z. B. Flap-Substratspezifität, pH-, Salzionen- und Temperaturoptimum) besitzt. Dann ist eine Kombination der mindestens einen geeigneten Polymerase, mit oder ohne intrinsische FEN-Aktivität, mit mindestens einer oder mehr FEN vorzuziehen. Die Kombination der vorgenannten Enzyme ist abhängig von der zu untersuchenden Probe und/oder den weiteren Komponenten des erfindungsgemäßen Reaktionsgemisches im Einzelfall anzupassen.

In einer weiteren Ausführungsform des erfindungsgemäßen Bestätigungsverfahrens umfasst das vorgenannte Reaktionsgemisch zusätzlich mindestens ein FEN-Verstärker-Oligonukleotid (z. B. Can_ENH1, Can_ENH2, Can_ENH3 und/oder Can_ENH4), insbesondere zur Steigerung der intrinsischen FEN-Aktivität einer Polymerase, vorzugsweise der *Taq* DNA Polymerase. Bevorzugt wird durch die Zugabe der FEN-Verstärker-Oligonukleotide (ENH₁₋ₙ) die intrinsische FEN-Aktivität einer Polymerase, vorzugsweise der *Taq* DNA Polymerase, zumindest quantitativ und optional qualitativ gesteigert. Das mindestens eine FEN-Verstärker-Oligonukleotid (ENH₁₋ₙ) überlappt mit seiner Sequenz am 3'-Ende um mindestens eine Base mit der Zielsequenz-spezifischen 3'-Sequenz an der 5'-Bindungsstelle der mindestens einen FEN-Sonde, wie es auch in Fig. 1 dargestellt ist. In ENH₁₋ₙ ist n eine ganze Zahl. Vorzugsweise größer gleich 1 bis kleiner gleich 50. Insbesondere ist n eine ganze Zahl und vorzugsweise gleich 2, 3, 4, 5, 6, 7, 8, 9 oder 10. Beispiele für erfindungsgemäße FEN-Verstärker-Oligonukleotide sind Can_ENH1, Can_ENH2, Can_ENH3 und/oder Can_ENH4.

Ein weiterer Gegenstand der vorliegenden Erfindung ist ein Reaktionsgemisch, insbesondere zur Verwendung in dem erfindungsgemäßen Bestätigungsverfahren, umfassend
- mindestens zwei Zielsequenz-spezifische Primer (P1, P2, P₁₋ₙ), die zur Amplifikation mindestens einer nachzuweisenden Zielsequenz geeignet sind,
- mindestens eine markierte Zielsequenz-spezifische Flap-Endonukleasesonde (FEN-Sonde FEN1, FEN₁₋ₙ), wobei die mindestens eine FEN-Sonde umfasst
   - eine Zielsequenz-spezifische 3'-Sequenz, welche komplementär zu einem Sequenzabschnitt der mindestens einen Zielsequenz innerhalb einer Region ist, die von dem mindesten ersten Primer (P1) und dem mindestens zweiten Primer (P2) auf der Zielsequenz begrenzt wird,
   - am 3' -Ende der Zielsequenz-spezifischen 3'-Sequenz eine Schutzgruppe und
   - eine Zielsequenz-unspezifischen 5'-Sequenz, welche an ihrem 5'-Ende mit einem Hapten eines spezifischen Haptenpaares markiert ist, wobei nach Spaltung der mindestens einen FEN-Sonde, das mindestens eine erhaltene 5'-Spaltprodukt (S₁₋ₙ) mit dem Hapten markiert ist und als Primer zur Amplifikation der mindestens einen artifiziellen Matrizensequenz dient, und
- mindestens eine artifizielle Matrizensequenz, welche komplementäre Sequenzen zu dem mindestens einen 5'-Spaltprodukt (S1-n) der mindestens einen FEN-Sonde.

Es wird ein Reaktionsgemisch offenbart, insbesondere zur Verwendung in dem erfindungsgemäßen Bestätigungsverfahren, umfassend
- mindestens zwei Zielsequenz-spezifische Primer (P1, P2, P₁₋ₙ), die zur Amplifikation mindestens einer nachzuweisenden Zielsequenz geeignet sind,
- mindestens eine erfindungsgemäße markierte Zielsequenz-spezifische, insbesondere intermolekulare, Flap-Endonukleasesonde (FEN-Sonde FEN1, FEN₁₋ₙ), und
- mindestens eine artifizielle Matrizensequenz, insbesondere artifizielle Nukleinsäuresequenz,
- optional mindestens einen weiteren Primer (M1), der an den komplementären Strang der mindestens einen artifiziellen Matrizensequenz bindet, und/oder
- optional mindestens ein FEN-Verstärker-Oligonukleotid (ENH₁₋ₙ).

Somit umfasst eine Ausführungsform des erfindungsgemäßen Reaktionsgemisches, insbesondere zum Nachweis einer Zielsequenz im Rahmen einer in-vitro-Diagnostik,
- zusätzlich mindestens eine nachzuweisende Zielsequenz, insbesondere innerhalb einer biologischen Probe, vorzugsweise humanen Probe
- mindestens zwei erfindungsgemäße Zielsequenz-spezifische Primer (P1, P2, P₁₋ₙ),
- mindestens eine erfindungsgemäße Zielsequenz-spezifische markierte FEN-Sonde (synonym = FEN-Sonde) (siehe Fig. 1),
- mindestens eine artifizielle Matrizensequenz, insbesondere artifizielle Nukleinsäuresequenz, und
- mindestens ein FEN-Verstärker-Oligonukleotid (z. B. Can_ENH1, Can_ENH2, Can_ENH3 und/oder Can_ENH4).

In einer weiteren bevorzugten Ausführungsform des erfindungsgemäßen Bestätigungsverfahrens beträgt der Gehalt der mindestens einen nachzuweisenden Zielsequenz, vorzugsweise DNA, größer gleich 2 Kopien in dem erfindungsgemäßen Reaktionsgemisch, insbesondere in einem Reaktionsgemisch enthaltend mindestens ein FEN-Verstärker-Oligonukleotid (ENH₁₋ₙ). Vorzugsweise beträgt der Gehalt der mindestens einen nachzuweisenden Zielsequenz größer gleich 5, größer gleich 10, größer gleich 15, größer gleich 20, größer gleich 25, größer gleich 50, größer gleich 100 Kopien und besonders bevorzugt bis jeweils kleiner gleich 1000 Kopien, jeweils als Endkonzentration bezogen auf das gesamte Reaktionsgemisch.

In einer weiteren bevorzugte Ausführungsform des erfindungsgemäßen Bestätigungsverfahrens beträgt der Gehalt der mindestens einen nachzuweisenden Zielsequenz, vorzugsweise DNA, größer gleich 10 Kopien in dem erfindungsgemäßen Reaktionsgemisch, insbesondere in einem Reaktionsgemisch ohne FEN-Verstärker-Oligonukleotide (ENH₁₋ₙ). Vorzugsweise beträgt der Gehalt der mindestens einen nachzuweisenden Zielsequenz größer gleich 15, größer gleich 20, größer gleich 25, größer gleich 35, größer gleich 50, größer gleich 75, größer gleich 100, größer gleich 150, größer gleich 250 Kopien und besonders bevorzugt bis jeweils kleiner gleich 1000 Kopien, jeweils als Endkonzentration bezogen auf das gesamte Reaktionsgemisch.

Die vorgenannten Untergrenzen wurden ausgehend von der, in den Beispielen 1-3, real eingesetzten DNA-Menge [fg] der *C. albicans* Zielsequenz ermittelt, wobei 2 fg einer doppelsträngigen DNA ca. 10 Kopien einer nachzuweisenden Zielsequenz entsprechen (Umrechnung siehe Beispiel 3).

Insbesondere ist die erfindungsgemäß nachzuweisende Zielsequenz, vorzugsweise DNA, besonders bevorzugt eine doppelstränge DNA, eine Zielsequenz, welche in multiplen Kopien pro Zelle vorliegt, umfassend mitochondriale DNA (mtDNA), rDNA, SINE (short interspersed nuclear element, *Alu*-Familie) und/oder MIR (mammalian-wide interspersed repeats).

In einer weiteren Ausführungsform des erfindungsgemäßen Bestätigungsverfahrens liegt die mindestens eine nachzuweisende Zielsequenz, insbesondere umfassend RNA, DNA, cDNA und/oder rDNA, vorzugsweise eine Zielsequenz, welche in multiplen Kopien pro Genom vorliegt, innerhalb einer biologischen Probe vor, insbesondere einer humanen Probe. Besonders bevorzugt umfasst die humane Probe mindestens eine nachzuweisende Zielsequenz
- einer Flüssigkeit umfassend extrazelluläre Kreislaufflüssigkeiten, wie Blut, Plasma, Serum und/oder Lymphe, Verdauungssäfte, wie Speichel, Magensaft, Saft der Pankreas und/oder Galle, Sekrete,
- eines Exkrets, wie Schweiß, Urin, Kot, Ejakulat, Vaginalsekrete, Tränenflüssigkeit, Nasensekret und/oder Muttermilch,
- einer weiteren Flüssigkeit oder Absonderung, wie Fruchtwasser, Ohrenschmalz, Hirnwasser und/oder Eiter, und/oder
- eines Gewebes, Nägel, Haare und/oder Knochenbestandteile.

Zum Nachweis des Einflusses der FEN-Verstärker-Oligonukleotide (ENH₁₋ₙ) auf die Stärke des Signals, wurden erfindungsgemäß unterschiedliche Kombinationen getestet. Die hier beschriebenen Versuche und Ergebnisse, wie in Beispiel 1 und Tabelle 2 dargestellt, wurden aufgrund beschränkter Ressourcen mittels eines elektrophoretischen Verfahrens analysiert. Diese Ergebnisse gelten entsprechend für Haptenpaar markierte Matrizenamplifikate und die dazu erforderlichen erfindungsgemäßen Ausführungsformen zur Verwendung mit immunchromatographischen Verfahren, vorzugsweise NALFT. Eine ausgewählte Ausführungsform zu NALFT ist in Beispiel 2, Fig. 4, sowie in Beispiel 3, Fig. 5, dargestellt.

In einer bevorzugten Ausführungsform umfasst das erfindungsgemäße Reaktionsgemisch mindestens eine markierte FEN-Sonde (FEN1) und mindestens ein FEN-Verstärker-Oligonukleotid (z. B. Can_ENH1, Can_ENH2, Can_ENH3 und/oder Can_ENH4). Ein erfindungsgemäßes Beispiel ist in Tabelle 2 dargestellt. Überraschenderweise wurde bereits durch die Kombination von nur einer FEN-Sonde, z. B. Can_FEN2, und nur einem FEN-Verstärker-Oligonukleotid, z. B. Can_ENH2, im Bestätigungstest mit einem elektrophoretischen Detektionsverfahren ein 5-fach stärkeres Signal (4954 RFU) im Vergleich zu einem Bestätigungstest mit nur einer FEN-Sonde oder zwei unterschiedlich markierten FEN-Sonden, z. B. Can_FEN2 und Can_FEN1 (900/931 RFU) erzielt. Eine entsprechende Signalsteigerung ist im immunchromatographischen Verfahren, vorzugsweise NALFT zu erwarten.

Somit resultiert im erfindungsgemäßen Bestätigungsverfahren die Zugabe des mindestens einen FEN-Verstärker-Oligonukleotids (z. B. Can_ENH1, Can_ENH2, Can_ENH3 und/oder, Can_ENH4) überraschend in einer mindestens 5-fachen Verstärkung des Signals des mindestens einen erhaltenen Matrizensequenzamplifikats, vorzugsweise im immunchromatographischen Verfahren.

Ausgewählte Kombinationen der FEN-Sonden, FEN-Verstärker-Oligonukleotiden und des weiteren Primers M1 (siehe Tabelle 3) aus Beispiel 1 wurden in Beispiel 3 in Abhängigkeit des Gehalts der nachzuweisenden Zielsequenz (DNA-Ausgangsmaterial einer biologischen Probe) näher untersucht. Das erfindungsgemäße Reaktionsgemisch umfassend eine FEN-Sonde (FEN1) und einen weiteren Primer (M1) sowie das erfindungsgemäße Reaktionsgemisch umfassend eine FEN-Sonde (FEN1), einen weiteren Primer (M1) und ein FEN-Verstärker-Oligonukleotid (ENH1), welche jeweils bei 50 pg DNA-Ausgangsmaterial in Beispiel 1 hervorragende Signale (5900 RUF bzw. 4213 RFU) in der Kapillargelelektrophorese lieferten, ermöglichen auch bei geringen DNA-Mengen von lediglich 20 fg bzw. 2 fg bereits visuell auswertbare Nachweise im immunchromatographischen Verfahren anhand von distinkten Banden im Detektionsfeld des LFT (Beispiel 3, Fig. 5: Reaktionsgemisch 3 bzw. 11 und 18).

Ein FEN-Verstärker-Oligonukleotid (ENH₁₋ₙ) hybridisiert mit der Zielsequenz unmittelbar stromaufwärts der Zielsequenz-spezifischen 3'-Sequenz einer FEN-Sonde. Dabei überlappt das 3'-Ende des FEN-Verstärker-Oligonukleotids (ENH₁₋ₙ) mit dem Anteil der FEN-Sonde, welcher mit der Zielsequenz exakt zur Doppelhelix gepaart ist, um mindestens ein Nukleotid. Die mit der FEN-Sonde überlappende 3'-Sequenz des FEN-Verstärker-Oligonukleotids muss dabei mit der Zielsequenz nicht zwingend hybridisieren, sondern kann einen ungepaarten 3'-Flap ausbilden (Kaiser *et al.* 1999). Diese Anordnung führt zu einer deutlichen Erhöhung der Spaltaktivität der intrinsischen FEN einer Polymerase, vorzugsweise der *Taq* DNA Polymerase. Für andere FEN-Enzyme sind andere strukturelle Eigenschaften der FEN-Verstärker-Oligonukleotide denkbar.

In einer weiteren Ausführungsform des erfindungsgemäßen Bestätigungsverfahrens ist das 5'-Ende der Zielsequenz-unspezifischen 5'-Sequenz der mindestens eine FEN-Sonde mit einem Detektor-Hapten oder einem zur Matrizensequenz sequenzspezifisches Hapten eines spezifischen Haptenpaares markiert (siehe Tabelle 1).

In einer weiteren Ausführungsform des erfindungsgemäßen Bestätigungsverfahren umfasst das erfindungsgemäße und vorbeschriebene Reaktionsgemisch ferner mindestens zwei, vorzugsweise unterschiedlich, markierte FEN-Sonden (FEN1 und FEN2, FEN₁₋ₙ), die jeweils eine Zielsequenz-spezifische 3'-Sequenz umfassen und mindestens einen weiteren Primer (M1), welcher komplementär zu einem Sequenzabschnitt eines Gegenstrangs der mindestens einen artifizielle Matrizensequenz ist (siehe Beispiel 1: FEN1+FEN2, FEN2+M1, Beispiel 2, B: FEN1+FEN2; Beispiel 3: FEN2 + M1).

In einer weiteren Ausführungsform des erfindungsgemäßen Bestätigungsverfahrens liegt das andere Hapten des spezifischen Haptenpaares am 5'-Ende der Zielsequenz-unspezifischen 5'-Sequenz der mindestens zweiten FEN-Sonde oder des mindestens einen weiteren Primers (M1) als Markierung vor (siehe Tabelle 1: Can_FEN2, WB127FD).

In einer weiteren Ausführungsform des erfindungsgemäßen Bestätigungsverfahrens
- erfolgt die Amplifikation der mindestens einen artifiziellen Matrizensequenz mit mindestens einem markierten 5'-Spaltprodukt (S1, S₁₋ₙ) der mindestens einen FEN-Sonde und mindestens einem weiteren markierten 5-Spaltprodukt (S2, S₁₋ₙ) der mindestens zweiten FEN-Sonde oder mit mindestens einem markierten 5'-Spaltprodukt der mindestens einen FEN-Sonde und mindestens einem markierten weiteren Primer (M1),
- erfolgt die Markierung der mindestens einen artifiziellen Matrizensequenz mit dem spezifischen Haptenpaar während der Amplifikation und
- es wird mindestens ein markierter Doppelstrang des mindestens einen Matrizensequenzamplifikats erhalten, welcher am jeweiligen Strang ein Hapten des spezifischen Haptenpaar aufweist.

Vorzugsweise erfolgt die Markierung mit einem Hapten des 5'-Endes der Zielsequenz-unspezifischen 5'-Sequenz der mindestens einen FEN-Sonde und dem zweiten Hapten einer zweiten FEN-Sonde oder eines weiteren Primers (M1).

Somit ist erfindungsgemäß ein Reaktionsgemisch bevorzugt, welches umfasst
- mindestens eine nachzuweisende Zielsequenz,
- mindestens zwei erfindungsgemäße Zielsequenz-spezifische Primer (P1, P2, P₁₋ₙ),
- mindestens zwei erfindungsgemäße FEN-Sonden, die mit ihren jeweiligen sequenzspezifischen 3'-Sequenzen komplementär zu einem unterschiedlichen Sequenzabschnitte der mindestens einen Zielsequenz sind, wie es beispielhaft in Fig. 1 gezeigt ist, und
- mindestens eine artifizielle Matrizensequenz,
wobei die mindestens zwei FEN-Sonden mit jeweils einem Haptenpaar eines spezifischen Haptenpaares markiert sind oder nur eine FEN-Sonde mit einem Hapten des spezifischen Haptenpaares markiert ist.

Vorzugsweise umfasst das vorgenannte Reaktionsgemisch mindestens ein FEN-Verstärker-Oligonukleotid (z. B. Can_ENH1, Can_ENH2, Can_ENH3 und/oder Can_ENH4).

Folglich hybridisieren im erfindungsgemäßen Bestätigungstest die mindestens zwei FEN-Sonden an ihre jeweiligen an der Zielsequenz komplementären Sequenzen. Durch Spaltung der FEN-Sonden werden mindestens zwei 5'-Spaltprodukte (S1, S2, S₁₋ₙ) erhalten, die als Primer zur Amplifikation der mindestens einen artifiziellen Matrizensequenz dienen. Dadurch wird ein verstärkt messbares Signal gemessen, dass den Nachweis der amplifizierten Zielsequenz anzeigt.

Ein spezifisches Haptenpaar besteht stets aus einem Detektor-Hapten und einem Matrizensequenz sequenzspezifischen Hapten. Spezifische Haptenpaare sind dem Fachmann bekannt oder können kommerziell bei Anbietern bezogen werden und umfassen beispielsweise das Streptavidin-Biotin-System, aber auch Antikörper-Antigen- bzw. Hapten-Paare. Insbesondere sind als Detektor-Haptene Biotin oder ein Biotinanalogon wie Iminobiotin oder Desthiobiotin geeignet sowie o-Nitrophenol, Peptide und/oder Fluorophore. Unter Biotinanalogon ist dabei jedes mit Streptavidin bindefähiges Molekül zu verstehen. Neben Antikörpern und Streptavidin sind dem Fachmann auch andere proteinogene Rezeptoren (*englisch* binding scaffolds) sowie Amptamere (aus RNA oder DNA bzw. chemische Derivate davon wie z. B. L-Ribose, Peptid-Nukleinsäuren, LNA, Thio- und Dithiophoshate u.a.m.) als Bildungspartner für Haptene bekannt.

Spezifische Haptenpaare sind funktionell so aufgebaut, dass sie zum einen das Substrat, vorliegend die erfindungsgemäße Matrizensequenz, erkennen und zum anderen ein Signal unter Einwirkung des Detektor-Haptens ermöglichen, wenn eine Sequenzerkennung stattgefunden hat.

Die Detektion des Signals erfolgt durch Messung der erhaltenen Matrizenamplifikate bevorzugt anhand eines im sichtbaren Licht sichtbaren Farbsignals, eines emittierenden Fluoreszenzsignals, eines Quantenpunkts (*englisch* quantum dot) und/oder mittels so genannter Up-converting Phosphor Reporter (Hampl *et al.* 2001).. Andere Detektionsprinzipien sind dem Fachmann bekannt. Als Signalgeber werden bevorzugt mit Hapten-Rezeptoren konjugierte kolloidale Partikel (*synomym* Detektionskolloid) wie kolloidales Gold oder mit Farbstoffen hochdicht gefärbte Nanopartikel aus Latex und Silikaten (z. B. DCN Diagnostics, Carlsbad, US-CA) sowie Zellulose (z. B. NanoAct™ Zellulosenanopartikel, Asahi Kasei Fibers Corp., Osaka, JP) verwendet. Fluoreszenzsignale können während der Amplifikation auch direkt durch die 5'-Spaltprodukte (S₁₋ₙ), welche das Fluorophor als Detektor-Hapten tragen, auf das mindestens eine Matrizenamplifikat übertragen werden.

Das Fluoreszenzsignal umfasst mindestens ein Fluorophor welches Licht einer bestimmten Wellenlänge von größer gleich 400 nm bis kleiner gleich 800 nm emittiert. Fluoreszenzfarbstoffe sind dem Fachmann bekannt und können in Kombination mit der erfindungsgemäßen FEN-Sonde frei gewählt werden. Unter Berücksichtigung des verwendeten Detektionsverfahrens sind gerätespezifische Beschränkungen bei Design der FEN-Sonden zu berücksichtigen.

Fluoreszenzfarbstoffe umfassen Uranin, Rhodamine, Fluorescein, DAPI, Phycoerythrin Cumarine, Allophycocyanin, 7-Aminoactinomycin, Indocyaningrün / ICG, Calcein, Cumarin, Cyanine, Chinin-Hydrogensulfat, Fluorescein arsenical helix binder, GFP - **G**reen **F**luorescent **P**rotein, Quadraine (Quadratsäurefarbstoffe) auf Basis von N,N-Dialkylanilinen, 1,3,2-Dioxaborine (Komplexe von Borsäurederivaten mit 1,3-Dicarbonylverbindungen), Safranin, und Stilben. Weitere geeignete Fluoreszenzfarbstoffe bzw. Fluorophore kennt der Fachmann und wählt diese aus den verfügbaren Fluorophoren von aktuellen Anbietern aus, wie biomers.net GmbH, Atto-tec GmbH, Dyomics GmbH und Themo Fischer Scientific - Molecular Probes.

In einer weiteren Ausführungsform des erfindungsgemäßen Bestätigungsverfahren umfasst das Reaktionsgemisch mindestens zwei artifizielle Matrizensequenzen, welche sich in der Sequenz und/oder Sequenzlänge unterscheiden und welche jeweils komplementäre Sequenzen zu mindestens einem 5'-Spaltprodukt (S₁₋ₙ) der jeweiligen FEN-Sonde oder zu mindestens einem weiteren Primer (M1) umfassen. Nach Amplifikation können sie die Matrizenamplifikate in der Sequenz, Sequenzlänge und/oder Konformation sowie optional in der Markierung unterscheiden.

Somit umfasst das erfindungsgemäße Reaktionsgemisch in einer Ausführungsform
- mindestens eine nachzuweisende Zielsequenz,
- mindestens zwei Zielsequenz-spezifische Primer (P1, P2, P₁₋ₙ), die zur Amplifikation der mindestens einen Zielsequenz geeignet sind,
- mindestens zwei erfindungsgemäße FEN-Sonden, die mit ihren jeweiligen sequenzspezifischen 3'-Sequenzen komplementär zu einem unterschiedlichen Sequenzabschnitte der mindestens einen Zielsequenz sind, wie es beispielhaft in Fig. 1 gezeigt ist, wobei das 5'-Ende der Zielsequenz-unspezifischen 5'-Sequenz der mindestens ersten FEN-Sonde mit einem Detektor-Hapten oder einem zur Matrizensequenz sequenzspezifisches Hapten eines spezifischen Haptenpaares markiert ist und
- mindestens zwei artifizielle Matrizensequenzen, welche jeweils komplementäre Sequenzen zu mindestens einem 5'-Spaltprodukt (S1, S2, S₁₋ₙ) der jeweiligen FEN-Sonde (FEN1 und FEN2) umfassen oder
- mindestens zwei artifizielle Matrizensequenzen, wovon jeweils eine Matrizensequenz eine komplementäre Sequenzen zu mindestens einem weiteren Primer (M1) und die andere zu einer der zwei FEN-Sonden umfasst.

In einer weiteren Ausführungsform liegt das andere Hapten des spezifischen Haptenpaares am 5'-Ende der Zielsequenz-unspezifischen 5'-Sequenz der mindestens zweiten FEN-Sonde vor, vorzugsweise unterscheiden sich die FEN-Sonden untereinander in Sequenz, Sequenzlänge und/oder Markierung.

In einer weiteren Ausführungsform des erfindungsgemäßen Bestätigungsverfahrens umfasst das Reaktionsgemisch, insbesondere zur Steigerung der Amplifikation, zumindest quantitativ und optional qualitativ, der mindestens einen artifiziellen Matrizensequenz,
- mindestens eine erfindungsgemäße FEN-Sonde, in der vorbeschriebenen Art, und mindestens einen weiteren Primer (M1),
- mindestens eine erfindungsgemäße FEN-Sonde (siehe oben), mindestens ein FEN-Verstärker-Oligonukleotid (z.B. Can_ENH1, Can_ENH2, Can_ENH3 und/oder Can_ENH4), wie oben bereits beschrieben, und mindestens einen weiteren Primer (M1), oder
- mindestens zwei erfindungsgemäße FEN-Sonden, wie oben beschrieben, mindestens ein FEN-Verstärker-Oligonukleotid (ENH₁₋ₙ) und mindestens einen weiteren Primer (M1), oder
- mindestens zwei erfindungsgemäße FEN-Sonden, mindestens zwei FEN-Verstärker-Oligonukleotid (ENH₁₋ₙ) und mindestens einen weiteren Primer (M1), oder
- mindestens zwei erfindungsgemäße, vorzugsweise unterschiedliche, FEN-Sonden und mindestens zwei FEN-Verstärker-Oligonukleotide (Fig. 4, C) Can_ENH1 und Can_ENH2; D) Can_ENH3 und Can_ENH4).
wobei der mindestens weitere Primer (M1) komplementär zu mindestens einem Sequenzabschnitt des Gegenstrang der mindestens einen artifizielle Matrizensequenz ist und die mindestens zweite FEN-Sonde eine von der ersten FEN-Sonde abweichende Zielsequenz-spezifische 3'-Sequenz aufweist, welche komplementär zu einer Sequenz innerhalb einer Region der mindestens einen Zielsequenz ist, die von dem mindesten ersten Primer (P1) und dem mindestens zweiten Primer (P2) begrenzt wird und dessen Spaltprodukt (S2) sich vom Spaltprodukt (S1) der ersten FEN-Sonde unterscheidet und komplementär zum Gegenstrang der mindestens einen artifizielle Matrizensequenz ist. Das erste Spaltprodukt (S1) ist entsprechend komplementär zur 3'-Sequenz in (3→ 5') der Matrizensequenz. Ferner umfasst jede der vorgenannten Alternativen mindestens ein spezifisches Haptenpaar zur Markierung der mindestens einen Matrizensequenz, wobei das zur Matrizensequenz sequenzspezifische Hapten und Detektor-Hapten auf jeweils unterschiedlichen FEN-Sonden und/oder weiteren Primern (M1) angeordnet ist (siehe Tabelle 1). Ein Beispiel ist in Fig. 1 gezeigt.

Überraschenderweise wurde in der vorliegenden Erfindung herausgefunden, dass jede der vorgenannten Kombinationen zu einer signifikanten Verstärkung des Signals des mindestens einen erhaltenen Matrizenamplifikats führt, jeweils im Vergleich zum Signal, welches mit einem Reaktionsgemisch mit einer oder zwei FEN-Sonden im erfindungsgemäßen Bestätigungsverfahren erhalten wird (siehe Tabelle 2). Entsprechende Ergebnisse sind mit dem immunchromatographischen Verfahren zu erwarten und ebenfalls für Haptenpaar markierte Matrizenamplifikate und die dazu erforderlichen erfindungsgemäßen Ausführungsformen zur Verwendung mit immunchromatographischen Verfahren, vorzugsweise NALFT. Eine ausgewählte Ausführungsform zu NALFT ist in Beispiel 2, Fig. 4, dargestellt. Weitere Ausführungsformen des erfindungsgemäßen Bestätigungsverfahrens sind in Beispiel 3, Fig. 5, gezeigt.

In einer bevorzugten Ausführungsform führt im Reaktionsgemisch die Kombination des mindestens einen FEN-Verstärker-Oligonukleotids (ENH₁₋ₙ) und eines weiteren Primer (M1) im erfindungsgemäßen Bestätigungsverfahren zu einer signifikanten Verstärkung des Detektionssignals, vorzugsweise zu mindestens einem 30-fach verstärkten Signal, des mindestens einen erhaltenen, insbesondere markierten, Matrizensequenzamplifikats im Vergleich zur Verwendung von einer oder zwei FEN-Sonden wie exemplarisch für ein elektrophoretisches Verfahren gezeigt (Tabelle 2, 900 bzw. 931 RFU).

Die mit den vorgenannten Ausführungsformen erzielten Signale sind in Tabelle 2 gezeigt. Die Kombination aus einer markierten FEN-Sonde und einem weiterem Primer (M1) zeigt mit einem Wert von 5900 RFU (Tabelle 2) im Vergleich zum Bestätigungstest mit nur einer oder zwei FEN-Sonden (900/931 RFU) eine signifikante Verstärkung des Signals, ein mehr als 6-fach stärkeres Signal - bestätigt in Beispiel 3, Fig. 4. Die Kombination aus einer FEN-Sonde, einem FEN-Verstärker-Oligonukleotid (ENH₁₋ₙ) und einem weiterem Primer (M1) führt mit einem Werte von 42130 RFU zu einer weiteren Steigerung des Signals des Matrizenamplifikats (Tabelle 2) im Vergleich zum Bestätigungstest mit nur einer oder zwei FEN-Sonden (900/931 RFU) um mehr als das 46-fache - bestätigt in Beispiel 3, Fig. 5. Ähnliche Werte werden mit der Kombination von zwei FEN-Sonden (FEN1 und FEN2), einem FEN-Verstärker-Oligonukleotid und einem weiteren Primer M1 erzielt (30747 RFU).

Eine Kombination aus zwei FEN-Sonden und zwei FEN-Verstärker-Oligonukleotiden führt mit Werten von 6511 bzw. 3500 RFU zu einer bis zu 7-fachen Verstärkung des Signals des, vorzugsweise Fluoreszenz markierten, Matrizenamplifikats, welches in einer Kapillargelelektrophorese detektiert wird. Der entsprechende Versuchsaufbau mit vergleichbar guten Ergebnissen in einem immunchromatographischen Verfahren ist in Beispiel 2 und Fig. 4 gezeigt.

Alle vorgenannten Ausführungsformen weisen die Ausführbarkeit des Bestätigungstest zur immunchromatographischen Detektion im Sinne der Erfindung nach, der in einem kollektiven und kontinuierlichen Reaktionsansatz für die Amplifikation der Zielsequenz und der Amplifikation und optional Markierung der Matrizensequenz (Eintopfverfahren) stattfindet. Abhängig von der Kombination der FEN-Sonde(n) mit einem oder mehr FEN-Verstärker-Oligonukleotiden und/oder eines weiteren Primers (M1) wird eine signifikante Steigerung des Signals erzielt. Diese überraschenden Ergebnisse belegen die erfinderische Lösung zur gestellten Aufgabe. Diese Ausführungsformen, insbesondere die Kombination mit mindestens einem FEN-Verstärker und/oder einem weiteren Primer (M1), bieten variable Lösungen für einen kontinuierlichen Reaktionsansatz für die Amplifikation und Bestätigungstest von Zielsequenzen, insbesondere aus humanen Proben wie Blut, Plasma, Serum, Knochen und/oder Gewebe und reduziert das Risiko der Kontamination mit Fremd-DNA, RNA, Proteine, Peptide und/oder Chemikalien. Dabei hat das Signal eine hervorragende Qualität und Stärke, sodass ein vereinfachtes und verbessertes Eintopf-Diagnostikverfahren einschließlich des Bestätigungstest bereitgestellt wird. Insbesondere ein Diagnostikverfahren, welches die Anforderungen gemäß Richtlinie MIQ-1 2011 für Nukleinsäure-Amplifikations-Techniken und/oder die Richtlinie der Bundesärztekammer B3 (Rili BÄK-B3) für den direkten Nachweis und Charakterisierung von Infektionserregern erfüllt sowie auch jeweils die Anforderungen der jeweiligen Novellierungen der Richtlinie erfüllt.

In einer weiteren Ausführungsform des erfindungsgemäßen Bestätigungsverfahrens hybridisieren die Zielsequenz-spezifischen 3'-Sequenz der mindestens ersten und/oder der mindestens zweiten FEN-Sonde an ihre jeweils komplementäre, insbesondere einzelsträngige, Zielsequenz desselben Moleküls oder an zwei unterschiedlichen Zielsequenzmolekülen, sodass Komplexe gemäß Fig. 1 und 2 erhalten werden, worin beide FEN-Sonden am selben Zielsequenzmolekül hybridisieren oder zwei unterschiedliche Anordnungen. In einer ersten Anordnung hybridisiert die erste Zielsequenz-spezifische 3'-Sequenz an ein erstes Zielsequenzmolekül und in der zweiten Anordnung hybridisiert die zweite Zielsequenz-spezifische 3'-Sequenz an ein zweites Zielsequenzmolekül. Vorzugsweise sind die zwei Zielsequenzmoleküle unterschiedlich und besonders bevorzugt sind es mindestens zwei unterschiedliche Zielsequenzen in einer Probe, insbesondere in einer humanen Probe gemäß obiger Definition.

In einer weiteren Ausführungsform des erfindungsgemäßen Bestätigungsverfahrens, welches vorzugsweise ein Multiplex-Verfahren ist, umfasst das Reaktionsgemisch
- mindestens zwei oder mehr nachzuweisende Zielsequenzen, vorzugsweise unterschiedliche in einer, insbesondere humanen, Probe enthaltende Zielsequenzen,
- eine Kombination aus mindestens zwei oder mehr FEN-Sonden, welche sich in ihrer Sequenz, Sequenzlänge und/oder Markierung untereinander unterscheiden, die jeweils insbesondere komplementär zu den unterschiedlichen Zielsequenzen sind und
- mindestens zwei oder mehr unterschiedliche artifizielle Matrizensequenzen, welche jeweils eine komplementäre Sequenz zu den mindestens zwei 5'-Spaltprodukten (S1, S2, S₁₋ₙ) der mindestens zwei FEN-Sonden umfasst, insbesondere ist jede ein Nachweis für jeweils eine Zielsequenz und
wobei im Bestätigungsverfahren je Zyklus der Vervielfältigungsreaktion
- mindestens zwei oder mehr unterschiedliche markierte artifizielle Matrizensequenzamplifikate erhalten werden,
- jede amplifizierte Zielsequenz durch jeweils mindestens ein markiertes artifizielles Matrizensequenzamplifikat bestätigt wird und
- die mindestens zwei oder mehr markierten artifiziellen Matrizensequenzamplifikate in einem immunchromatographischen Verfahren distinkt detektiert und quantifiziert werden.

Bei zwei oder mehreren nachzuweisende Zielsequenzen, werden zwei oder mehr FEN-Sonden eingesetzt, welche sich zumindest in der 3'-Sequenz zueinander unterscheiden. Ferner unterscheiden sich die 5'-Enden der FEN-Sonden ebenfalls zueinander.

Insbesondere umfasst das erfindungsgemäße Multiplex-Kit, vorzugsweise in räumlich getrennter Anordnung oder als gebrauchsfertiges Gemisch, Puffersystem, Nukleotide, Salze etc. und alle weiteren für eine erfolgreiche PCR erforderlichen Komponenten. Diese sind dem Fachmann bekannt und/oder werden vom Gerätehersteller der zur Amplifikation und/oder Detektion eingesetzten Geräte vorgeben. Vorzugsweise wird das erfindungsgemäße Multiplex-Kit gebrauchsfertig für die Diagnostik einer, insbesondere humanen, Probe bereitgestellt.

Ein weiterer Gegenstand der vorliegenden Erfindung ist eine Zusammensetzung umfassend eine Kombination aus mindestens zwei unterschiedlichen markierten Zielsequenz-spezifischen Flap-Endonukleasesonde (FEN-Sonde FEN1, FEN₁₋ₙ), wobei jede FEN-Sonde jeweils umfasst
- eine Zielsequenz-spezifische 3'-Sequenz, welche komplementär zu einem Sequenzabschnitt der mindestens einen Zielsequenz innerhalb einer Region ist, die von dem mindesten ersten Primer (P1) und dem mindestens zweiten Primer (P2) auf der Zielsequenz begrenzt wird, wobei die mindestens zwei FEN-Sonden sich zumindest in der 3'-Sequenz und/oder 3'-Sequenzlänge zueinander unterscheiden,
- am 3'-Ende der Zielsequenz-spezifischen 3'-Sequenz eine Schutzgruppe insbesondere als Polymeraseblocker, und
- eine Zielsequenz-unspezifischen 5'-Sequenz, und
mindestens zwei artifizielle Matrizensequenzen, welche sich zumindest in der Sequenzlänge um mindestens 10 Basenpaare und/oder der Sequenz und optional der Markierung zueinander unterscheiden, wobei jedes der 5'-Spaltprodukte (S1, S2, S₁₋ₙ) der FEN-Sonden eine komplementäre Sequenz zu jeweils einem Sequenzabschnitt einer artifizielle Matrizensequenz oder ihres Gegenstrangs aufweist.

Daher ist ein weiterer Gegenstand der vorliegenden Erfindung ein flüssiges Gemisch umfassend
- einen PCR-Puffer, insbesondere mit einem geeigneten pH-Wert,
- Nukleotide,
- mindestens eine (FEN1) oder mehrere FEN-Sonden (FEN₁₋ₙ), welche sich in ihrer Sequenz, Sequenzlänge und/oder Markierung untereinander unterscheiden, und jede FEN-Sonde umfasst
   - eine Zielsequenz-spezifischen 3'-Sequenz, welche komplementär zu einem Sequenzabschnitt mindestens einer Zielsequenz innerhalb einer Region ist, die von mindestens einem ersten Primer (P1) und mindestens einem zweiten Primer (P2) auf der Zielsequenz begrenzt wird,
   - am 3'-Ende der Zielsequenz-spezifische 3'-Sequenz eine Schutzgruppe, insbesondere als Polymeraseblocker, vorzugsweise fehlt die 3'-OH-Gruppe, und
   - eine Zielsequenz-unspezifischen 5'-Sequenz, und
- mindestens eine oder mehr unterschiedliche artifizielle Matrizensequenzen, welche sich in ihrer Sequenz und/oder Sequenzlänge unterscheiden und welche jeweils komplementäre Sequenzen zu mindestens einem 5'-Spaltprodukt (S1, S₁₋ₙ) der mindestens einen FEN-Sonde oder zu mindestens einem weiteren Primer umfassen, und
- optional Zusätze und Additive, die dem Fachmann bekannt sind.

Das erfindungsgemäße Bestätigungsverfahren sowie das erfindungsgemäße Multiplex-Kit sind bevorzugt ein Verfahren und/oder Kit zur Diagnose und Bestätigung der Diagnose von Bakterien, Parasiten, Pilzen und/oder Viren. Insbesondere zum Nachweise von *Chlamydia, Streptokokkus, Legionella, Listeria,* MRSA, *Mykobakterium, Salmonella, Toxoplasma, Candida,* Hepatitis, HIV, Influenza, Varizella-Zoster, Parvovirus und/oder Enteroviren.

Somit ist ein weiterer Gegenstand der vorliegenden Erfindung ein Bestätigungsverfahren, wobei insbesondere das Reaktionsgemisch mindestens zwei markierte, vorzugsweise unterschiedliche, FEN-Sonden (FEN1, FEN2, FEN₁₋ₙ) oder mindestens eine FEN-Sonde und mindestens einen weiteren Primer (M1) umfasst,
- die Amplifikation der mindestens einen artifiziellen Matrizensequenz mit mindestens einem 5'-Spaltprodukt (S1, S₁₋ₙ) der mindestens einen FEN-Sonde (FEN1) erfolgt und mindestens einem weiteren 5'-Spaltprodukt der mindestens zweiten FEN-Sonde (FEN2) oder mindestens einem weiteren Primer (M1),
- die Markierung der mindestens einen artifiziellen Matrizensequenz während der Amplifikation erfolgt, wobei das Matrizensequenzamplifikat mit einem spezifischen Haptenpaar markiert wird und
- mindestens ein markierter Doppelstrang des mindestens einen Matrizensequenzamplifikats erhalten wird, welcher am jeweiligen Strang ein Hapten des spezifischen Haptenpaar aufweist und
- insbesondere das Haptenpaar markierte Matrizensequenzamplifikat wird an einer Festphase distinkt detektiert. Insbesondere innerhalb einer definierten Detektionszone einer Festphase, vorzugsweise innerhalb einer Detektionszone eines Lateralflow-Teststreifens wie in Fig.4 gezeigt.

Unter einem amplifikatspezifischen (synonym = spezifischen) Haptenpaar, insbesondere für einen NALFT, wird die Kombination von zwei Haptenen mit einem spezifischen doppelsträngigen DNA-Amplifikat verstanden. Dabei sind die jeweiligen Haptene des spezifischen Haptenpaares über die stabile DNA-Doppelhelix spezifisch miteinander verbunden (oder "verbrückt") und bewirken durch ihre Bindung, ein messbares Signal.

Der wesentliche Unterschied des erfindungsgemäßen Bestätigungsverfahrens zum Stand der Technik ist, dass das erhaltene mindestens eine Haptenpaar-markierte artifizielle Matrizensequenzamplifikat die Bestätigung (synonym = Nachweis) der mindestens einen nachzuweisenden amplifizierten Zielsequenz ist. Kann das Haptenpaar-markierte artifizielle Matrizensequenzamplifikat auf irgendeine Weise nachgewiesen bzw. gemessen werden, so war der Bestätigungstest erfolgreich.

Daher ist ein weiterer Gegenstand der vorliegenden Erfindung das mindestens eine markierte artifizielle Matrizensequenzamplifikat, welches durch die mindestens zwei 5'-Spaltprodukte (S1, S2, S₁₋ₙ) der mindestens zwei FEN-Sonden markiert wurde oder welches durch das 5'-Spaltprodukt der mindestens einen FEN-Sonde und den mindestens einen markierten weiteren Primer (M1) markiert wurde, insbesondere als Bestätigung der mindestens einen amplifizierten Zielsequenz.

Daher ist ein weiterer Gegenstand der vorliegenden Erfindung mindestens ein Haptenpaarmarkiertes artifizielles Matrizensequenzamplifikat, insbesondere erhalten oder erhältlich in einem erfindungsgemäßen Bestätigungsverfahren.

In einer bevorzugten Ausführungsform des erfindungsgemäßen Bestätigungsverfahrens wird das mindestens eine mit einem Haptenpaar-markierte artifizielle Matrizensequenzamplifikat, insbesondere erhalten aus der vorgeschalteten Amplifikation, mittels eines immunchromatographischen Verfahrens detektiert, vorzugsweise mittels eines Nukleinsäure-Lateral-Flow (NALF) immunchromatographisches Verfahrens.

Das Haptenpaar-markierte artifizielle Matrizensequenzamplifikat kann unmittelbar einem Detektionsverfahren zugeführt werden, oder zu einem späteren Zeitpunkt ortsunabhängig detektiert werden. Somit kann das erhaltene Haptenpaar-markierte artifizielle Matrizensequenzamplifikat gelagert werden. Das erfindungsgemäße Bestätigungsverfahren kann am Point-of-Need durchgeführt werden und die Detektion an einem Ort mit den entsprechenden Ressourcen (z. B. Verfügbarkeit eines Thermocylers oder isothermalen Prozessors sowie des NALF-Teststreifens) erfolgen.

Vorzugsweise wird im erfindungsgemäßen Bestätigungsverfahren dem mindestens einen Haptenpaar-markierten artifiziellen Matrizensequenzamplifikat unmittelbar ein geeigneter Puffer hinzugefügt und das erhaltene Gemisch unmittelbar dem immunchromatographischen Verfahren zugeführt, insbesondere mit einer Festphase in Kontakt gebracht. Vorzugsweise wird unmittelbar ein Lateral-Flow-Laufpuffer hinzugefügt und das erhaltene Gemisch auf ein Probenapplikationsbereich eines Lateral-Flow Teststreifens aufgebracht.

In einer bevorzugten Ausführungsform des erfindungsgemäßen Bestätigungsverfahrens wird das mindestens eine Haptenpaar-markierte artifizielle Matrizensequenzamplifikat über ein Signal detektiert, welches von einer fluoreszierenden und/oder von einer im sichtbaren Licht messbaren Verbindung abgegeben wird. Dabei initiiert das Haptenpaar-markierte artifizielle Matrizensequenzamplifikat die jeweilige Reaktion mindestens einer Präverbindung zu der jeweils messbaren Verbindung. Vorzugsweise wird die mindestens eine Präverbindung zu einer Signal abgebenden Verbindung prozessiert, insbesondere enzymatisch gespalten und/oder durch Einwirkung einer Lichtquelle angeregt.

In einer weiteren Ausführungsform des erfindungsgemäßen Bestätigungsverfahrens umfasst das Reaktionsgemisch
- größer eins bis kleiner gleich 10 unterschiedliche FEN-Sonden, welche entsprechend mit jeweils einem Hapten der größer eins bis kleiner 10 unterschiedlicher spezifischer Haptenpaare markiert sind und
- größer eins bis kleiner gleich 10 unterschiedliche artifizielle Matrizensequenzen.

In einer besonderen Ausführungsform des erfindungsgemäßen Bestätigungsverfahrens wird eine Kombination aus größer eins bis kleiner gleich 10, vorzugsweise zwei, drei, vier, fünf, sechs, sieben, acht, neun oder zehn, unterschiedlicher mit jeweils einem spezifischen Haptenpaar-markierter artifizielle Matrizensequenzamplifikate im einem kollektiven und kontinuierlichen Reaktionsgemisch bestätigt und mittels eines immunchromatographischen Verfahrens simultan und distinkt an einer Festphase detektiert. Vorzugsweise werden größer gleich 2 bis kleiner gleich 10 Haptenpaar-markierter artifizielle Matrizensequenzamplifikate innerhalb einer definierten Detektionszone einer Festphase, vorzugsweise eines Lateralflow-Teststreifens, detektiert. Dabei unterscheiden sich die Haptenpaar-markierte artifizielle Matrizensequenzamplifikate in der Kombination unterschiedlicher spezifischer Haptenpaare, der Sequenz, Sequenzgröße und/oder Konformation der Haptenpaar-markierten artifiziellen Matrizensequenzamplifikate. Durch eine geeignete Wahl unterschiedlicher spezifischer Haptenpaare und artifizieller Matrizensequenzen können in einer Detektionszone eines Teststreifens bis zu 10 Zielsequenzen bestätigt werden.

Das erfindungsgenmäße Bestätigungsverfahren, bevorzugt unter Verwendung einer Festphase zur Detektion des Signals des mindestens einen Haptenpaar-markierten Matrizensequenzamplifikats, umfasst die Schritte
- Interaktion des mindestens einen mobilen, insbesondere in einem Puffer befindlichen, Haptenpaar-markierten Matrizensequenzamplifikats über das Detektor-Hapten mit einem Rezeptormolekül des Detektor-Haptens, wobei das Rezeptormolekül an ein Detektionskolloid als signalerzeugende Präverbindung konjugiert ist und gleichzeitig als Instant-Zubereitung (Instant-Zubereitung umfasst Füllstoffe und/oder Stabilisatoren usw,) in einem vordefinierten Depotbereich (Konjugatzone) einer Festphase anhaftet,
- Alternativ kann es sich beim Detektor-Hapten auch um ein Fluorophor handeln, welches direkt von einem Fluoreszenzreader nachgewiesen werden kann
- Erhalt eines mobilen Matrizensequenzamplifikat/Rezeptormolekül/Detektionssingalverbindungs-Komplexes
- Interaktion des mindestens einen mobilen vorgenannten Komplex über das noch freie sequenzspezifische Hapten mit einem dafür spezifischen immobilisierten Rezeptormolekül und
- Detektion eines messbaren Signals, insbesondere in einer Detektionszone (synonym = Detektionsfeld) einer Festphase, vorzugsweise eines Teststreifens, wie in Fig. 4 gezeigt.

Die Ausführbarkeit des erfindungsgemäßen Verfahrens unter Verwendung einer Festphase im Detektionsverfahren, vorzugsweise eines Lateralflow-Teststreifen, ist in Beispiel 2 und 3 beschrieben und in Fig. 4 und 5 gezeigt. Es wurde am oberen Rand der Detektionszone eine distinkte Bande detektiert, welche das Haptenpaar markierte Matrizensequenzamplifikat repräsentiert und damit das Zielsequenzamplifikat bestätigt. Damit ist die Funktionalität und Ausführbarkeit der vorliegenden Erfindung ebenfalls hervorragend für Verfahren unter Verwendung einer Festphase im Detektionsverfahren, vorzugsweise eines Lateralflow-Teststreifens, belegt. Durch die geeignete Wahl der FEN-Verstärker-Oligonukleotide (Can_ENH1, Can_ENH2, Can-ENH3 und/oder Can-ENH4) wird eine stärker sichtbare Bande detektiert (Fig. 4 C und D; Fig. 5 Reaktionsgemische 8-11 und 15-18). Bei Einsatz von mehr als zwei artifiziellen Matrizensequenzamplifikaten und geeigneten spezifischer Haptenpaare können unterhalb der in Fig. 4 oder 5 sichtbaren Bande weitere Zielsequenzamplifikate anhand darunter detektierbarer Banden distinkt detektiert und bestätigt werden.

Ein weiterer Gegenstand der vorliegenden Erfindung ist ein Multiplex-Kit, insbesondere zur Verwendung im erfindungsgemäßen Bestätigungsverfahren, umfassend eine Kombination aus
- mindestens einer markierten (FEN1) oder mehr markierten FEN-Sonden (FEN₁₋ₙ), welche sich, insbesondere in ihrer Sequenz, Sequenzlänge und/oder Markierung, untereinander unterscheiden, und jede FEN-Sonde umfasst
   - eine Zielsequenz-spezifische 3'-Sequenz, welche komplementär zu einem Sequenzabschnitt mindestens einer Zielsequenz innerhalb einer Region ist, die von mindestens einem ersten Primer (P1) und mindestens einem zweiten Primer (P2) auf der Zielsequenz begrenzt wird,
   - am 3'-Ende der Zielsequenz-spezifische 3'-Sequenz eine Schutzgruppe, insbesondere als Polymeraseblocker, vorzugsweise fehlt die 3'-OH-Gruppe, und
   - eine Zielsequenz-unspezifischen 5'-Sequenz, welche an ihrem 5'-Ende mit einem Detektor-Hapten oder einem zur Matrizensequenz sequenzspezifisches Hapten eines spezifischen Haptenpaares markiert ist und wobei nach Spaltung der mindestens einen FEN-Sonde das mindestens eine erhaltene 5'-Spaltprodukt (S1-n) mit einem Hapten markiert ist und als Primer zur Amplifikation der mindestens einen artifiziellen Matrizensequenz dient,
- mindestens eine oder mehr unterschiedliche artifizielle Matrizensequenzen, welche sich in ihrer Sequenz, Sequenzlänge und/oder Konformation unterscheiden und welche jeweils komplementäre Sequenzen zu mindestens einem 5'-Spaltprodukt (S1, S₁₋ₙ) der mindestens einen FEN-Sonde oder zu mindestens einem weiteren Primer (M1) umfassen.

In einer weiteren Ausführungsform des erfindungsgemäßen Multiplex-Kits umfasst dieses mindestens zwei markierte FEN-Sonden (FEN1 und FEN2, FEN₁₋ₙ) und/oder mindestens eine markierte FEN-Sonde und mindestens einen weiteren markierten Primer (M1), wobei als Markierung jeweils ein Hapten eines spezifischen Haptenpaares vorliegt.

In einer weiteren Ausführungsform des erfindungsgemäßen Multiplex-Kits liegt das andere Hapten des spezifischen Haptenpaares am 5'-Ende der Zielsequenz-unspezifischen 5'-Sequenz der mindestens zweiten FEN-Sonde oder an dem mindestens weiteren Primer (M1) als Markierung vor, (siehe z.B. Tabelle 1).

Vorzugsweise umfasst das erfindungsgemäße Multiplex-Kit das vorbeschriebene Reaktionsgemisch, insbesondere als gebrauchsfertige Mischung zur Verwendung in dem erfindungsgemäßen Bestätigungsverfahren und alle weiten für eine PCR erforderlichen Zusätze wie z. B. Puffersystem, Nukleotide, Salze, etc., die dem Fachmann bekannt sind.

Ein weiterer Gegenstand der vorliegenden Erfindung ist die Verwendung des erfindungsgemäßen Reaktionsgemisches, wie oben definiert, und/oder die Verwendung des erfindungsgemäßen Multiplex-Kits , wie oben definiert, jeweils in dem erfindungsgemäßen Verfahren zur Bestätigung mindestens einer amplifizierten Nukleinsäure-Zielsequenz (Zielsequenz) während einer Vervielfältigungsreaktion in einem kollektiven und kontinuierlichen Reaktionsansatzes.

In einer Ausführungsform des erfindungsgemäßen Bestätigungsverfahrens sind die Haptene angeordnet,
- nach einer Alternative ein Hapten am 5'-Ende der Zielsequenz-unspezifischen 5'-Sequenz der mindestens einen FEN-Sonde FEN1 und das andere Hapten an mindestens einem weiteren Primer (M1) (siehe Tabelle 1), oder
- nach einer anderen Alternative jeweils ein Hapten auf jeweils einer ersten und einer zweiten FEN-Sonde (FEN1 und FEN2) (siehe Tabelle 1),
und diese markieren jeweils gemeinsam das mindestens eine Matrizensequenzamplifikat mit dem spezifischen Haptenpaar und
insbesondere zum Erhalt mindestens eines Haptenpaar markierten artifizielle Matrizensequenzamplifikats und insbesondere zur Detektion des vorgenannten Matrizenamplifikats an einer Festphase.

### Beschreibung der Figuren (Fig.):

**Fig. 1****: Relative Anordnung der verwendeten PCR-Primer, FEN-Sonden, FEN-Verstärker-Oligonukleotide und zusätzlichen Primer.** A) Die relative Bindungsstellen der PCR-Primer (Can_Set003_SP11, Can_Set002_ASP1), FEN-Sonden (Can_FEN1, Can_FEN2) und FEN-Verstärker-Oligonukleotide (Can_ENH1-4) in Bezug auf die 18s rDNA-Region von *C. albicans* sind dargestellt. Der 5'→3' Strang entspricht der Genbank Zugangsnummer AY497754. X steht für Hapten 2, Y steht für 6-Carboxyfluorescein oder Hapten 1. B) Die zielsequenzunabhängigen 5'-Sequenzbereiche (gestrichelte Pfeile) der FEN-Sonden Can_FEN1 und Can_FEN2 binden nach ihrer Abspaltung an die artifizielle Matrizensequenz Alpha 1. Die DNA-Sequenz des unmarkierten (WB127F), optional mit einem Hapten markierten (WB127FD), Primers WB127 ist identisch zum abgespalteten 5'-Sequenzbereich der FEN-Sonde Can_FEN1. Die Figuren sind nicht maßstabsgerecht dargestellt. Polymeraseblocker (3'-C3-carbon spacer) sind als vollflächige Karos dargestellt. 3'-Nukleotide der FEN-Verstärker-Oligonukleotide (ENH₁₋ₙ), welche mit den zielsequenz-spezifischen Bereichen der FEN-Sonden überlappen, sind als offene Kreise dargestellt. Pfeile verweisen auf Oligonukleotide, welche als Primer fungieren können.
**Fig. 2****: Hybridisierung der FEN-Sonden (Can_FEN1, 2) und FEN-Verstärker-Oligonukleotide (Can_EHN1** - **4) mit ihren Zielsequenzen.** A) Die Sequenzen von Can_FEN1 und den korrespondierenden FEN-Verstärker-Oligonukleotiden Can_ENH1 und Can_ENH3 ist dargestellt. B) Die Sequenzen von Can_FEN2 und den korrespondierenden FEN-Verstärker-Oligonukleotiden Can_ENH2 und Can_ENH4 ist dargestellt. Die Zielsequenz entspricht dem Gegenstrang der 18s rDNA-Region von *C. albicans* mit der Genbank Zugangsnummer AY497754. X, Unmarkiert oder Hapten 2; Y, 6FAM, 6-Carboxyfluorescein oder Hapten 1; Spacer 3, Polymeraseblocker 3'-C3 carbon spacer.
**Fig. 3****: Analyse eines artifiziellen Amplifikats, welches in Abhängigkeit des Spaltprodukts der FEN-Sonde Can_FEN2 gebildet wurde, mittels dem Applied Biosystems® 3500 Genetic Analyzer.** Die PCR bestand aus 50 pg genomischer DNA von *C. albicans,* den PCR-Primern Can_Set003_SP11 und Can_Set002_ASP1, der 5'-6FAM-markierten FEN-Sonde Can_FEN2, dem FEN-Verstärker-Oligonukleotid Can_ENH2, dem zusätzlichen unmarkierten Primer WB127F und der artifiziellen Matrizensequenz Alpha 1. Weitere Details siehe Text. Ein Aliquot von 1 µL einer 1:20 Verdünnung des PCR-Amplifikats wurde analysiert. A) 6FAM-Analysekanal mit dem Amplifikationsprodukt von Alpha 1. B) Längenstandard im Analysekanal BTO. RFU, relative Fluoreszenzeinheiten.
**Fig. 4****: Nachweis der PCR-Amplifikate mittels Lateralflow (LF) Immunchromatographie-Teststreifen (LFT).** Der funktionelle Aufbau der LFT ist am linken Rand erläutert. Ein Pfeil am rechten Rand markiert die spezifische Detektionslinie. Die PCR wurde wie im Beispiel 1 beschrieben mit genomischer DNA von *C. albicans,* dem Primerpaar Can_Set003_SP11 und Can_Set002_ASP1 sowie den 5'-Hapten-markierten FEN-Sondenpaar Can_FEN1 und Can_FEN2 und der artifiziellen Matrizensequenz Alpha 1 durchgeführt. A) Negativkontrolle ohne genomische DNA. B) Komplette PCR ohne zusätzliche FEN-Verstärker-Oligonukleotide. C) Komplette PCR, zusätzlich mit den FEN-Verstärker-Oligonukleotiden Can_ENH1 und Can_ENH2. D) Komplette PCR, zusätzlich mit den FEN- Verstärker-Oligonukleotiden Can_ENH3 und Can_ENH4.
**Fig. 5****: Nachweis der PCR-Amplifikate mittels LFT in Abhängigkeit des Gehalts der nachzuweisenden Zielsequenz.** Der funktionelle Aufbau der LFT ist bereits in Fig. 4 dargestellt und erläutert, sodass in Fig. 5 ausschließlich die Detektionszone des jeweiligen LFT dargestellt ist. Die PCR wurde wie in Beispiel 1 beschrieben mit genomischer DNA von *C. albicans* als nachzuweisende Zielsequenz, dem Primerpaar (P1, P2) Can_Set003_SP11 und Can_Set002_ASP1, der 5'-Hapten 1-markierten FEN-Sonde Can_FEN2, den 5'-Hapten 2-markierten zusätzlichen Primer (M1) WB127FD und optional in Kombination mit einem FEN-Verstärker-Oligonukleotid Can_ENH1 oder Can_ENH4 und der artifiziellen Matrizensequenz Alpha 1 durchgeführt. Als Kontrollen wurden das vorgenannte Reaktionsgemisch ohne Zielsequenz (ZK), das vorgenannte Reaktionsgemisch ohne Matrizensequenz (MK) und das vorgenannte Reaktionsgemisch mit einer KlenTaq DNA Polymerase ohne FEN-Aktivität (FK) anstelle einer Taq DNA Polymerase eingesetzt.

Im Folgenden werden ausgewählte Bespiele zur Erzielung der erfindungsgemäßen Lösung erläutert, wobei die hier präsentierten Beispiele nicht beschränkend auszulegen sind.

### Beispiele

### Beispiel 1: Bestätigung eines PCR-Amplifikats unter Verwendung einer Flap-Endonuklease (FEN)-Sonde und einer artifiziellen Matrizensequenz mit dem Applied Biosystems® 3500 Genetic Analyzer

### Material und Methoden

**DNA-Aufreinigung aus *Candida albicans* DSM 1386:** Der Referenzstamm wurde über die DSMZ - Deutsche Sammlung für Mikroorganismen und Zellkulturen GmbH (Braunschweig, DE) bezogen. Hefezellen wurden bei 25 °C auf Sabouraud-Glucose-Agar mit Chloramphenicol (Bio-Rad Laboratories GmbH, München, DE) kultiviert. Die Zellen wurden mit einem sterilen Spatel geerntet und in steriler phosphatgepufferter Salzlösung (PBS, 137 mM NaCl, 2,7 mM KCl, 10 mM Na₂HPO₄, 1,8 mM KH₂PO₄, pH 7,4) resuspendiert. Die DNA-Aufreinigung erfolgte mit dem QIAamp® DNA Mini Kit (Qiagen GmbH, Hilden, DE) gemäß Herstellerangaben und folgender Veränderung: Die Proben wurden für den Zellaufschluss mit ATL-Puffer und Proteinase K des Herstellers versetzt und mindestens 12 h bei 50 °C inkubiert. Die aufgereinigte DNA wurde mittels UV-VIS-Spektroskopie unter Verwendung eines Eppendorf® BioPhotometer® (Eppendorf AG, Hamburg, Germany) quantifiziert.

**Entwurf und Synthese von PCR-Primern, FEN-Sonden, FEN-Verstärker-Oligonukleotiden und der universellen Matrizensequenz:** Ein Teil des 18s rDNA-Gens von *C. albicans* (Genbank Zugangsnummer AY497754) wurde als Zielsequenz ausgewählt. Eine 127 Basen lange artifizielle Matrizensequenz Alpha 1 wurde teilweise von der *lac*Zα-Sequenz des pUC19 Plasmids (Genbank Zugangsnummer L09137) abgeleitet. Die PCR-Primer, FEN-Sonden und FEN-Verstärker-Oligonukleotide wurden mit der Software Vector NTI® (Thermo Fisher Scientific Inc. - Life Technologies Div., Darmstadt, DE) and Mfold (Zuker 2003) entworfen. Die Primeranbindungstemperaturen (Tₐ) der FEN-Sonden (Can_FEN1 and Can_FEN2) und FEN-Verstärker-Oligonukleotide (Can_ENH1, Can_ENH2, Can_ENH3, Can_ENH4) wurden ähnlich den Entwurfsregeln für Hydrolysesonden mindestens 5 °C höher gewählt als die Tₐ der PCR-Primer (Heid *et al.* 1996).

Als FEN-Verstärker-Oligonukleotide wurden 3'-stromaufwärts der FEN-Sonden Oligonukleotide entworfen, welche am 3'-Ende mit ein (Can_ENH3, Can_ENH4) oder zwei (Can_ENH1, Can_ENH2) Nukleotiden zur zielsequenzspezifischen 5'-Bindungsstelle der FEN-Sonde überlappten und zusätzlich an ihrem 3'-Ende eine nicht-paarende Base enthielten (3'-Flap) (Lyamichev *et al.* 1993, Xu *et al.* 2001) (Figur 2).

Die relative Anordnung der PCR-Primer, FEN-Sonden und FEN-Verstärker-Oligonukleotide auf der Zielsequenz ist in Figur 1 dargestellt. Figur 2 zeigt die DNA-Sequenzen der FEN-Sonden und FEN-Verstärker-Oligonukleotide sowie ihre Bindungsstellen auf der Ziel-DNA.

Alle Oligonukleotide wurden in HPLC-gereinigter Qualität bei biomers.net GmbH (Ulm, DE) bezogen.

**Table 1: Primer, FEN-Sonden, FEN-Verstärker-Oligonukleotide und artifizielle Matrizensequenz. Die Primeranbindungstemperatur (Tₐ) wurde mit der Software Vector NTI® (Thermo Fisher Scientific Inc. - Life Technologies, Darmstadt, DE) unter Verwendung der Standardeinstellungen berechnet. Unterstrichende Sequenzen entsprechen den 5'-Enden der Flap-Endonuklease (FEN)-Sonden, welche nach der Abspaltung als PCR-Primer an die artifiziellen Matrizensequenz Alpha 1 oder deren Gegensequenz binden.**

| 6FAM, 6-Carboxyfluorescein; X = unmarkiert oder Hapten 2, Y= 6FAM oder Hapten 1, Spacer 3= 3'-C3 carbon spacer. | | |
|---|---|---|
| Name | Sequenz (5' → 3'-Ende) und Modifikation | Tₐ[°C] |
| Can_Set00 3_SP11 | GGTAGGATAGTGGCCTACCATGGTTT | 58,7 |
| Can_Set00 2_ASP1 | CCGACCGTCCCTATTAATCATTACGAT | 60,4 |
| Can_FEN1 | X-TTAACTATGCGGCATCAGAGCAGATTGGAGGGCAAGTCTGGT GCCAGC-Spacer 3 | 66,9 |
| Can_FEN2 | Y-CAACAGTTGCGCAGCCTGAATGCGTACTGGACCCAGCCGAG CC-Spacer 3 | 67,7 |
| WB127F | TTAACTATGCGGCATCAGAGCAGA | 57,8 |
| WB127FD | Hapten2- TTAACTATGCGGCATCAGAGCAGA | 57,8 |
| Can_F1 | AACCTTGGGCTTGGCTGGC | 58,6 |
| Can_ENH2 | CTTGGCTGGCCGGTCCATCTTTTTGAG | 68,0 |
| Can_ENH1 | TTGGAATGAGTACAATGTAAATACCTTAACGAGGAACAAG | 66,0 |
| Can_EN H3 | TTGGAATGAGTACAATGTAAATACCTTAACGAGGAACAG | 65,4 |
| Can_ENH4 | CTTGGCTGGCCGGTCCATCTTTTTGG | 66,8 |
| Alpha1 | TTAACTATGCGGCATCAGAGCAGATTGTACTGAGAGTGCACCAT ATGCGGTGTGAAATACCGCACAGATGCGTAAGGAGAAAATACC GCATCAGGCGCCATTCGCCATTCAGGCTGCGCAACTGTTG | nicht anwend -bar |

**Polymerasen-Kettenreaktion (PCR):** Die PCR wurde in einem Volumen von 25 µL durchgeführt und enthielt 1fachen REMA Puffer (mit Endkonzentrationen von 0,2 mM dNTPs und 1,5 mM MgCl₂; Biotype Diagnostic GmbH, Dresden, DE), 2,5 Units Multi *Taq2* DNA Polymerase (mit Hotstart-Funktion; Biotype Diagnostic GmbH, Dresden, DE), 10-50 pg chromosomaler DNA von *C. albicans,* 4 nM bis 4 µM artifizielle Matrizensequenz Alpha 1, 0,3 µM PCR-Primer und 0,3 µM FEN-Sonde Can_FEN2 mit 5'-6FAM-Markierung. Die unmarkierte FEN-Sonde Can_FEN2, FEN-Verstärker-Oligonukleotide und/oder Primer WB127F wurden optional ebenfalls in einer Endkonzentration von 0,3 µM eingesetzt (DNA-Sequenzen siehe Tabelle 1 und 2).

Nullkontrollen wurden ohne chromsomale DNA von *C. albicans* durchgeführt. Zusätzlich wurden Experimente mit *Klentaq*1, einer N-terminalen Deletionsvariante der *Taq* DNA Polymerase ohne FEN-Aktivität, durchgeführt (US5436149; DNA Polymerase Technology Inc., St. Louis, US-MO). Ein Eppendorf MasterCycler® ep Gradient Thermal Cycler (Eppendorf AG, Hamburg, DE) wurde verwendet. Der Temperaturwechsel bestand aus 4 min Hotstart-Aktivierung bei 96 °C und 35 Zyklen mit 30 s bei 96 °C, 60 s bei 60 °C und 60 s bei 72 °C. Zum Schluss wurde ein Verlängerungsschritt für 10 min bei 72 °C durchgeführt, und die Reaktionsansätze wurden anschließend bis zur weiteren Analyse bei 4 °C gelagert.

**Kapillargelelektrophorese unter Verwendung des Applied Biosystems® 3500 Genetic Analyzer (Thermo Fisher Scientific - Applied Biosystems Div., Foster City, US-CA):** Der Analyseautomat wurde mit dem 3500 POP-7™ Polymer (Performance Optimized Polymer) nach Herstellerangaben und mit folgenden Anpassungen verwendet: Die spektrale Kalibrierung des Geräts erfolgte mit dem virtuellen Filterset Any5Dye in Kombination mit dem Matrix-Standard BT5 (Fluoreszenzfarben 6FAM, BTG, BTY, BTR, BTO, für Blau, Grün, Gelb, Rot und Orange) (Biotype Diagnostic GmbH, Dresden, DE). Aliquote von 1 µL der PCR bzw. Verdünnungen davon wurden mit 12 µL HiDi Formamid (Thermo Fisher Scientific - Applied Biosystems Div., Foster City, US-CA) und 0,5 µL Längenstandard SST-BTO (Biotype Diagnostic GmbH, Dresden, DE) gemischt, für 3 min bei 95 °C inkubiert und anschließend bis zur elektrokinetischen Injektion (10.000 V, 5 s) bei Raumtemperatur im automatischen Probengeber des Geräts gelagert. Die Analyseeinheit des Analyseautomaten zeichnet relative Fluoreszenzeinheiten (RFU) über der Fragmentlänge (Basen, b) auf (siehe Figur 3). Die Gerätekonfiguration erlaubt maximal eine semi-quantitative Auswertung (bis zu 20 % Schwankungen zwischen den Injektionen gleicher Proben).

### Ergebnisse und Diskussion

Zunächst wurde die optimale Primeranbindungstemperatur Tₐ von 60 °C für die *C. albicans* PCR und dem PCR-Primerpaar Can_Set003_SP11 and Can_Set002_ASP1 in einem Tₐ-Gradienten zwischen 55 °C und 65 °C bestimmt. Eine spezifische Bande, die der kalkulierten Länge von 539 bp entsprach, konnte mittels Ethidiumbromidfärbung nach Agarosegelelektrophorese dargestellt werden (nicht gezeigt). Danach wurde die optimale Konzentration für die universelle DNA-Matrize Alpha 1 in PCRs ermittelt, welche zusätzlich zum PCR-Primerpaar 0,3 µM der FEN-Sonde Can_FEN2 mit 6FAM-Markierung am 5'-Ende und 4 nM bis 4 µM der artifiziellen Matrizensequenz Alpha1 enthielten. Die Ergebnisse wurden mittels Applied Biosystems® 3500 Genetic Analyzer ausgewertet.

Eine Verdünnung zwischen 20 nM bis 160 nM erbrachte gute Ergebnisse.

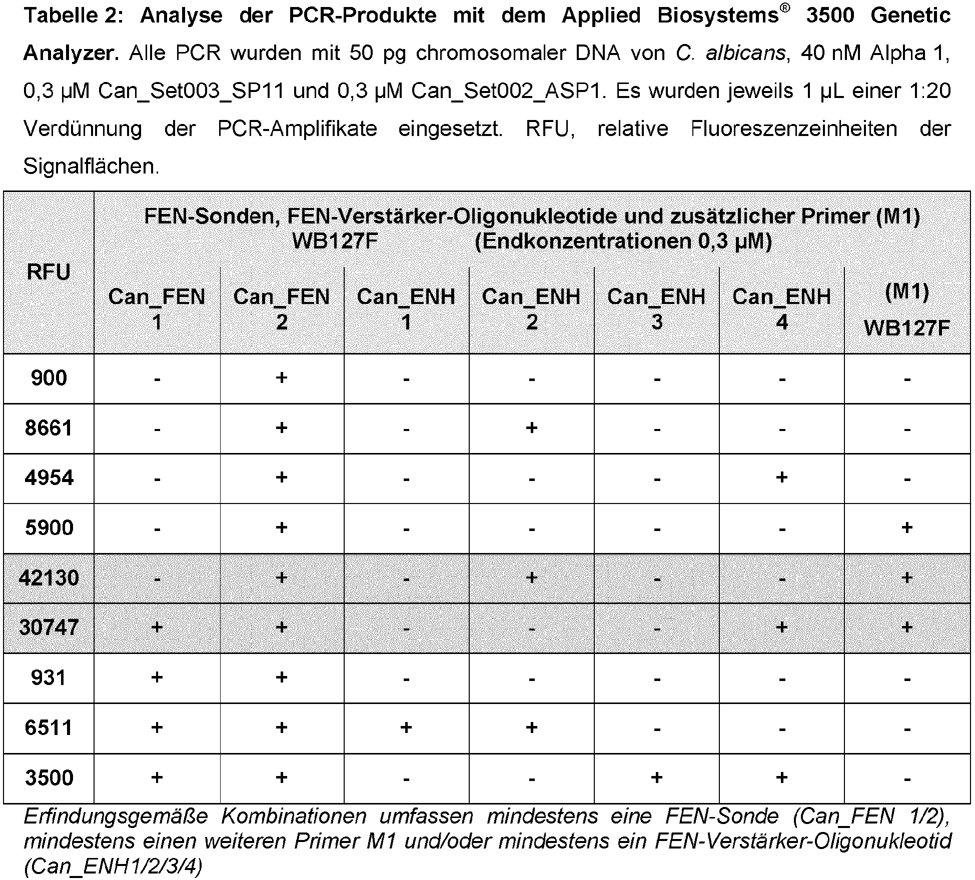

Anschließend wurden PCRs durchgeführt, welche stets 40 nM Alpha 1 und 0,3 µM der FEN-Sonde Can_FEN2 mit 5'-6FAM-Markierung enthielten. Zusätzlich wurden in bestimmten PCRs die unmarkierte FEN-Sonde Can_FEN1, FEN-Verstärker-Oligonukleotide und/oder der Primer WB127F, welcher an das 5'-Ende des Alpha 1-Gegenstrangs bindet (siehe auch Figur 1 und Tabelle 1), getestet. Die Ergebnisse sind in Tabelle 2 zusammengefasst. Das 127 bp Amplifikat, welches mit der artifiziellen Matrizensequenz Alpha 1 erwartet wurde, konnte bestätigt werden (Figur 3). Kontrollansätze ohne genomischer DNA oder mit der *KlenTaq*1 DNA Polymerase anstatt der *Taq* DNA Polymerase zeigten keine Amplifikate (nicht gezeigt). Die Amplifikation der artifiziellen Matrizensequenz Alpha 1 war somit abhängig von der FEN-Aktivität der *Taq* DNA Polymerase.

Wie in Tabelle 2 gezeigt konnte das Signal durch die Zugabe einer zweiten FEN-Sonde, deren 5'-Spaltprodukt an das 5'-Ende des Alpha 1-Gegenstrang bindet, nicht wesentlich gesteigert werden. Durch alleinige Zugabe der FEN-Verstärker Can_ENH2 oder Can_ENH4 konnten ca. 5,5-6,5fach höhere Signale erzielt werden. Die Zugabe eines FEN-Verstärkers und des Primers WB127F führte überraschenderweise zu ca. 34-46fach höheren Signalen.

### Beispiel 2: Bestätigung eines PCR-Amplifikats unter Verwendung von zwei FEN-Sonden, einer artifiziellen Matrizen und eines Lateralflow Immunchromatographie-Teststreifens

### Material und Methoden

**Chromsomale DNA und Oligonukleotide:** Die DNA-Aufreinigung aus *C. albicans* DSM 1386 und der Entwurf und die Synthese der Oligonukleotide wurde bereits im Beispiel 1 beschrieben (siehe auch Tabelle 1).

**PCR:** Die PCR wurde wie im Beispiel 1 beschrieben, jedoch unter Verwendung zweier FEN-Sonden, Can_FEN1 mit Hapten 2 am 5'-Ende markiert und Can_FEN2 mit Hapten 1 am 5'-Ende markiert, durchgeführt. Der weitere Primer (M1), WB127 (unmarkiert oder mit Hapten 2), wurde nicht eingesetzt. Primer, FEN-Sonden und FEN-Verstärker-Oligonukleotide wurden ebenfalls in Endkonzentrationen von 0,3 µM eingesetzt. Die Endkonzentration der artifiziellen Matrizensequenz Alpha 1 betrug 40 nM. PCR-Thermocycler und -Programm waren mit dem Beispiel 1 identisch.

**Nachweis von Nukleinsäureamplifikaten mittels Lateralflow (LF) Immunchromatographie-Teststreifen (LFT):** Es wurden Komponenten und Reagenzien eines Lohnherstellers verwendet. Die genaue chemische Zusammensetzung des LF-Laufpuffers und der Aufbau des LFTs (Haptene und deren Rezeptormoleküle) sind von Lohnerstellern in der Regel als Betriebsgeheimnisse geschützt und werden von diesem nicht offengelegt. Die Komponenten können jedoch zur Nacharbeitbarkeit zusammen mit kundenspezifischen haptenmarkierten Oligonukleotiden beim Hersteller bezogen werden (z. B. Amodia Bioservice GmbH, Braunschweig, DE). Der Aufbau des verwendeten Streifens ist in Figur 4 dargestellt. Er besitzt eine Detektionszone, welche mit haptenspezifischen Rezeptormolekülen (meist monoklonale Antikörper) in Linien funktionalisiert ist. Der verwendete LFT-Streifen besaß 5 verschiedene funktionalisierte Linien im Detektionsbereich. Verwendet wurde das Hapten 1, dessen Rezeptormolekül von der Eintauchzone am weitesten entfernt war. Der Konjugatbereich enthielt mit Stabilisatoren getrocknete Nanogoldpartikel, welche mit einem zweiten Rezeptormolekül (hier für Hapten 2) funktionalisiert waren. Die entsprechend markierten FEN-Sonden sind in Tabelle 1 und Figur 1 dargestellt.

Aliquote von 5 µL der PCR-Amplifikate wurden in einem 1,5 mL Reaktionsgefäß mit 100 µL LF-Laufpuffer gemischt. Der LFT wurde anschließend in den Reaktionsansatz getaucht und 20 min bei Raumtemperatur chromatographisch entwickelt. Die Ergebnisse wurden mit dem Auge ausgewertet.

### Ergebnisse und Diskussion

Die Ergebnisse sind in Figur 4 dargestellt. Im Vergleich zur Nullkontrolle konnte nur ein negatives bis schwachpositives Ergebnis erzielt werden wenn ausschließlich die beiden FEN-Sonden eingesetzt wurden. Der Zusatz von FEN-Verstärker-Oligonukleotiden führte zu einer eindeutigen Signalsteigerung wobei die Kombination von Can_ENH1 und Can_ENH2 zum besten Ergebnis führte.

### Beispiel 3: Bestätigung eines PCR-Amplifikats unter Verwendung einer Haptenmarkierten FEN-Sonde, in Kombination mit einem weiteren Hapten-markierte Primer (M1) und einem FEN-Verstärker-Oligonukleotid (ENH) mittels eines Lateralflow Immunchromatographie-Teststreifens (LFT)

### Material und Methoden

**Chromsomale DNA und Oligonukleotide:** Die DNA-Aufreinigung aus *C*. *albicans* DSM 1386 und der Entwurf und die Synthese der Oligonukleotide wurden bereits im Beispiel 1 beschrieben (DNA-Sequenzen siehe Tabelle 1).

**PCR:** Die PCR wurde analog zu der PCR aus Beispiel 1 durchgeführt, jedoch unter Verwendung der am 5'-Ende mit Hapten 1 markierten FEN-Sonde, Can_FEN2, und einem weiteren am 5'-Ende mit Hapten 2 markierten Primer (M1), WB127FD. Primer, FEN-Sonde und FEN-Verstärker-Oligonukleotid wurden ebenfalls in Endkonzentrationen von jeweils 0,3 µM eingesetzt. Die Endkonzentration der artifiziellen Matrizensequenz Alpha 1 betrug 40 nM. Es wurden unterschiedliche DNA-Mengen der nachzuweisenden Zielsequenz, 2000 fg, 200 fg, 20 fg und 2 fg chromosomale DNA von *C*. *albicans,* hinzugefügt. PCR-Thermocycler und Programm waren mit dem Beispiel 1 identisch.

Als Kontrollen dienten a) eine PCR ohne nachzuweisende Zielsequenz (ZK in Figur 5: Reaktionsgemische 6, 13 und 20), b) eine PCR ohne artifizielle Matrizensequenz (MK in Figur 5: Reaktionsgemische 5, 12, 19) und c) eine PCR mit 2,5 Units *KlenTaq1* DNA Polymerase (DNA Polymerase Technology Inc., St. Louis, US-MO) ohne FEN-Aktivität (FK in Figur 5: Reaktionsgemische 7, 14, 21).

**Nachweis von Nukleinsäureamplifikaten mittels Lateralflow (LF) Immunchromatographie- Teststreifen (LFT):** Der Nachweis erfolgte wie im Beispiel 2 beschrieben. Zusätzlich wurden die LFT mit dem LF-Lesegerät *opTrilyzer*® (opTricon GmbH, Berlin, DE) ausgewertet. Die Auswertesoftware *opTrilyzer*® *Data Viewer* des Lesegerätes ermittelt aus den Pixelintensitäten der Testlinienflächen seiner CCD-Kamera normierte Volumenwerte (relative Intensität der Linienflächen pro Pixel) (siehe Figur 5). Vom Hersteller wird eine gerätetechnische Nachweisgrenze (Limit of Quantification) von 10 normierten Volumenwerten angegeben.

### Ergebnisse und Diskussion

Die Ergebnisse sind in Figur 5 dargestellt. Die Kontrollen ohne Zielsequenz (ZK, Figur 5, Reaktionsgemisch 6, 13 und 20), ohne artifizielle Matrizensequenz Alpha 1 (MK, Figur 5, Reaktionsgemisch 5, 12 und 19) und mit *KlenTaq1* DNA Polymerase (FK, Figur 5, Reaktionsgemisch 7, 14 und 21) zeigten, wie erwartet, keine Banden. Dies belegt die Selektivität des erfindungsgemäßen Bestätigungsverfahrens.

Im Vergleich zu Beispiel 2 (siehe Fig. 4, LFT B), bei welchem mit zwei FEN-Sonden ohne FEN-Verstärker-Oligonukleotid und ohne weiteren Primer M1 keine Zielsequenz (Anfangsgehalt 50 pg) nachgewiesen werden konnte, wurden mit einer Kombination aus einer FEN-Sonde und einem weiteren markierten Primer (M1), WB127FD, auch ohne FEN-Verstärker-Oligonukleotid geringe Mengen der Zielsequenz erfolgreich nachgewiesen. (Figur 5, Reaktionsgemische 1-3).

Überraschenderweise wurde die Zielsequenz mit einem im Vergleich zu Beispiel 2 (50 pg) um Faktor 25, 250 und sogar 2500 geringeren Anfangsgehalt von 2000 fg, 200 fg und sogar nur 20 fg erfolgreich anhand einer distinkten Bande auf dem LFT nachgewiesen, welche mit dem menschlichen Auge sichtbar ist (Fig. 5, Reaktionsgemisch 1, 2 und 3).

Diese Ergebnisse bestätigen die in Beispiel 1 mittels des Applied Biosystems erzielten Ergebnisse für die Kombination aus einer FEN-Sonde und einem weiteren Primer M1 ohne FEN-Verstärker-Oligonukleotid (Tabelle 2, 5900 RFU) und zeigen, dass bereits eine FEN-Sonde für einen erfolgreichen und zweifelsfreien Nachweis geringer DNA-Mengen der Zielsequenz, auch mit dem bloßen Auge, ausreichend ist.

Die im erfindungsgemäßen Bestätigungsverfahren eingesetzten Reaktionsgemische der Beispiele 1, 2 und 3 sind in der nachfolgenden Tabelle 3 zusammengefasst.

**Tabelle 3: Übersicht der erfindungsgemäßen Reaktionsgemische (jeweils bezogen auf ein Gesamtvolumen von 25 µl)**

| **Reaktionsgemisch** | **Beispiel 1** | **Beispiel 2** | **Beispiel 3** |
|---|---|---|---|
| **Menge der nachzuweisende Zielsequenz (*C. albicans*)** | 50 pg | 50 pg | 2000/200/20/2/0 fg |
| **Primer (P1, P2)** | Can_Set003_SP11 | | |
| | Can_Set002_ASP1 | | |
| | Jeweils 0,3 µM | | |
| **FEN-Sonde(n) (FEN₁₋ₙ)** | Can-FEN1 | Can-FEN1 | Can-FEN2 |
| | Can-FEN2 | Can-FEN2 | ./. |
| | Jeweils 0,3 µM | Jeweils 0,3 µM | 0,3 µM |
| **Anzahl FEN₁₋ₙ** | 1 oder 2 | 2 | 1 |
| **Artifizielle** | Alpha1 | | |
| **Matrizensequenz** | 40 nM | | |
| **Weiterer Primer (M1)** | WB127F 0,3 µM | ohne | WB127FD 0,3 µM |
| **FEN-Verstärker-Oligonukleotid (ENH₁₋ₙ)** | Can_ENH2 oder | ./. | Can_ENH1 oder |
| | Can_ENH4 oder | ./. | Can_ENH4 |
| | Can_ENH/Can_ENH2 | Can_ENH1/Can_ENH2 | ./. |
| | oder | oder | |
| | Can_ENH3+ENH4 | Can_ENH3 + ENH4 | ./. |
| | Jeweils 0,3 µM | Jeweils 0,3 µM | Jeweils 0,3 µM |
| **Anzahl ENH₁₋ₙ** | 1 oder 2 | 0 oder 2 | 0 oder 1 |

Unter Zusatz eines FEN-Verstärker-Oligonukleotids (ENH1 oder ENH4) konnte eine Zielsequenz sogar mit einem Anfangsgehalt von nur 2 fg erfolgreich anhand einer distinkten Bande nachgewiesen werden (Figur 5, Reaktionsgemische 11 und 18). Damit erzielt das erfindungsgemäße Bestätigungsverfahren, dessen Ausführbarkeit bereits in Beispiel 1 und 2 gezeigt wurde, noch einmal eine im Vergleich zu Beispiel 2 (50pg zu 2 fg) um Faktor 25000 höhere Sensitivität.

Die Ergebnisse von Beispiel 3 (Fig. 5, Reaktionsgemische 8-11, 15-18) bestätigen die in Beispiel 1 mittels des Applied Biosystems erzielten Ergebnisse für die Kombination aus einer FEN-Sonde, einem weiteren Primer M1 und einem FEN-Verstärker-Oligonukleotid bei einem Anfangsgehalt der Zielsequenz von 50 pg (Tabelle 2, 42130 RFU) und belegen zusätzlich einen erfolgreichen und zweifelsfreien Nachweis sehr geringer Mengen der nachzuweisenden Zielsequenz (20 fg bzw. 2 fg) auch mit dem bloßen Auge unter Verwendung des vorgenannten Reaktionsgemisches.

Die visuelle Auswertung mit dem bloßen Auge sowie die Darstellung der distinkten Bande in der Detektionszone des LFT nach dem Einscannen des LFT belegt eindeutig die Funktionalität des erfindungsgemäßen Reaktionsgemisches und Bestätigungsverfahrens.

Eine Nachweisgrenze von 2 fg Ausgangsmaterial an nachzuweisender Zielsequenz von *C. albicans* konnte mittels eines für diagnostische Zwecke kalibrierten Lateralflow-Readers reproduzierbar gezeigt werden (Figur 5, Reaktionsgemisch 11 und 18; Cuttoff 20 normierte Volumenwerte).

*C. albicans* besitzt ein diploides Genom mit durchschnittlich 29,2 Mb (Megabasenpaare) (Hirakawa *et al.* 2015) und zuzüglich ca. 48 Kopien mitochondriale DNA (mtDNA) mit ca. 0,04 Mb (Fukuoh *et al.* 2014). Somit enthält eine Zelle von *C. albicans* ca. 31,1 Mb DNA. Da 1 fg doppelsträngiger DNA 0,978 Mb entsprechen (Dolezel *et al.* 2003), ergeben sich für die Hefezelle rechnerisch ca. 32 fg DNA-Gehalt pro Zelle. Pilze besitzen pro Genom ca. 20-200 Kopien von 18s rDNA Genen, welche als Zielsequenzen im Test verwendet wurden.

Somit lässt sich folgern, dass mit dem erfindungsgemäßen Verfahren unter Verwendung der *C. albicans* Zielsequenz mit multiplen Kopien pro Genom sicher eine Zelle und mit hoher Wahrscheinlichkeit mindestens 10 Kopien der nachzuweisenden Zielsequenz, welche die stochastische Untergrenze eines praktikablen Labortests ausmachen, nachgewiesen wurden.

Die vorstehende Umrechnung ist auf jedes Genom und damit für jede andere nachzuweisende Zielsequenz, vorzugsweise DNA, übertragbar. Folglich ist ein weiterer Gegensand der vorliegenden Erfindung ein Bestätigungsverfahren der hier beschriebenen Art, in dem der Gehalt der mindestens einen nachzuweisenden Zielsequenz, insbesondere DNA, vorzugsweise doppelstränge DNA, besonders bevorzugt einer Zielsequenzen, welche in multiplen Kopien pro Zelle vorlieget umfassend mtDNA, rDNA, SINE und/oder MIR, größer gleich 10 Kopien (entsprechend ca. 2 fg nachzuweisende Zielsequenz) im Reaktionsgemisch beträgt.

### Zitierte Literatur:

Dolezel J, Bartos J, Voglmayr H, Greilhuber J (2003). Nuclear DNA content and genome size of trout and human. Cytometry A 51:127-8.
Fukuoh A, Cannino G, Gerards M, Buckley S, Kazancioglu S, Scialo F, Lihavainen E, Ribeiro A, Dufour E, Jacobs HT (2014). Screen for mitochondrial DNA copy number maintenance genes reveals essential role for ATP synthase. Mol Syst Biol 10: 734.
Hampl J, Hall M, Mufti NA, Yao YM, MacQueen DB, Wright WH, Cooper DE (2001). Upconverting phosphor reporters in immunochromatographic assays. Anal Biochem 288:176-187.
Heid CA, Stevens J, Livak KJ, Williams PM (1996). Real time quantitative PCR. Genome Res 6, 986-994.
Hirakawa MP, Martinez DA, Sakthikumar S, Anderson MZ, Berlin A, Gujja S, Zeng Q, Zisson E, Wang JM, Greenberg JM, Berman J, Bennett RJ, Cuomo CA (2015). Genetic and phenotypic intra-species variation in Candida albicans. Genome Res 25: 413-25.
Hu J, Wang S, Wang L, Li F, Pingguan-Murphy B, Lu TJ, Xu F (2014). Advances in paperbased point-of-care diagnostics. Biosens Bioelectron 54: 585-597.
Kaiser MW, Lyamicheva N, Ma W, Miller C, Neri B, Fors L, Lyamichev VI (1999). A comparison of eubacterial and archaeal structure-specific 5'-exonucleases. J Biol Chem 274: 21387-21394.
Lyamichev V, Brow MA, Dahlberg JE (1993). Structure-specific endonucleolytic cleavage of nucleic acids by eubacterial DNA polymerases. Science 260: 778-783.
MIQ-1 (2011). Mikrobiologisch-infektiologische Qualitätsstandards (MiQ) - Nukleinsäure-Amplifikationstechniken (NAT) Richtlinien der DGHM in Zusammenarbeit mit BÄMI, DGP, DGPI, DMykG, DSTIG, DTG, DVV, ESGMD/ ESCMID, GfV, INSTAND, SGM (Reischl U et al., Urban & Fischer, München, ISBN-13 978-3-437-41535-5).
Rili-BÄK-B3 (2013). Richtlinie der Bundesärztekammer zur Qualitätssicherung laboratoriumsmedizinischer Untersuchungen (Rili-BÄK). Teil B3: Direkter Nachweis und Charakterisierung von Infektionserregern. Deutsches Ärzteblatt 110: A 575-A 582.
Xu Y, Potapova O, Leschziner AE, Grindley ND, Joyce CM (2001). Contacts between the 5' nuclease of DNA polymerase I and its DNA substrate. J Biol Chem 276, 30167-30177.
Zuker M (2003). Mfold web server for nucleic acid folding and hybridization prediction. Nucl Acids Res 31: 3406-3415.

## Patentansprüche

1. Bestätigung mindestens einer amplifizierten Nukleinsäure-Zielsequenz (Zielsequenz) während einer Vervielfältigungsreaktion in einem kollektiven und kontinuierlichen Reaktionsansatz enthaltend ein Reaktionsgemisch umfassend
- mindestens eine nachzuweisende Zielsequenz,
- mindestens zwei Zielsequenz-spezifische Primer (P1, P2, P₁₋ₙ), die zur Amplifikation der mindestens einen Zielsequenz geeignet sind,
- mindestens eine markierte Zielsequenz-spezifische Flap-Endonukleasesonde (FEN-Sonde FEN1, FEN₁₋ₙ), wobei die mindestens eine FEN-Sonde umfasst
• eine Zielsequenz-spezifische 3'-Sequenz, welche komplementär zu einem Sequenzabschnitt der mindestens einen Zielsequenz innerhalb einer Region ist, die von dem mindesten ersten Primer (P1) und dem mindestens zweiten Primer (P2) auf der Zielsequenz begrenzt wird,
• am 3' -Ende der Zielsequenz-spezifischen 3'-Sequenz eine Schutzgruppe und
• eine Zielsequenz-unspezifischen 5'-Sequenz, welche an ihrem 5'-Ende mit einem Hapten eines spezifischen Haptenpaares markiert ist, und
- mindestens eine artifizielle Matrizensequenz,
- mindestens einen weiteren Primer (M1), wobei der weitere Primer (M1) komplementär zu einem Sequenzabschnitt des Gegenstrangs der mindestens einen artifizielle Matrizensequenz ist und/oder
- mindestens ein FEN-Verstärker-Oligonukleotid (ENH₁₋ₙ)
das Bestätigungsverfahren umfasst je Zyklus der Vervielfältigungsreaktion die Schritte
- Hybridisierung der Zielsequenz-spezifischen 3'-Sequenz der mindestens einen FEN-Sonde an eine komplementäre Sequenz der mindestens einen nachzuweisenden Zielsequenz,
- Spaltung der mindestens einen FEN-Sonde,
- Erhalt von mindestens einem freien 5'-Spaltprodukt (S₁₋ₙ) umfassend jeweils die Zielsequenz-unspezifische 5'-Sequenz,
- Hybridisierung des mindestens einen 5'-Spaltprodukts (S₁₋ₙ) der mindestens einen FEN-Sonde an eine komplementäre Sequenz der mindestens einen artifiziellen Matrizensequenz,
- Amplifikation der mindestens einen artifiziellen Matrizensequenz mit dem mindestens einen 5'-Spaltprodukt (S₁₋ₙ) der mindestens einen FEN-Sonde und dem mindestens einen Primer (M1), und
- optional Markierung der mindestens einen artifiziellen Matrizensequenz während der Amplifikation durch das mindestens eine 5'-Spaltprodukt (S₁₋ₙ) der mindestens einen FEN-Sonde.

2. Bestätigungsverfahren nach Anspruch 1, wobei das 5'-Ende der Zielsequenz-unspezifischen 5'-Sequenz der mindestens einen FEN-Sonde mit einem Detektor-Hapten oder einem zur Matrizensequenz sequenzspezifisches Hapten eines spezifischen Haptenpaares markiert ist.

3. Bestätigungsverfahren nach Anspruch 1 oder 2, wobei der Gehalt der mindestens einen nachzuweisenden Zielsequenz größer gleich 2 Kopien im Reaktionsgemisch beträgt.

4. Bestätigungsverfahren nach einem der Ansprüche 1 bis 3, wobei die mindestens eine nachzuweisende Zielsequenz innerhalb einer biologischen Probe, vorzugsweise einer humanen Probe, vorliegt.

5. Bestätigungsverfahren nach einem der Ansprüche 1 bis 4, wobei das Reaktionsgemisch umfasst
- mindestens zwei markierte FEN-Sonden (FEN1 und FEN2, FEN₁₋ₙ), welche jeweils eine Zielsequenz-spezifische 3'-Sequenz umfassen.

6. Bestätigungsverfahren nach einem der Ansprüche 1 bis 5, wobei das andere Hapten des spezifischen Haptenpaares am 5'-Ende der Zielsequenz-unspezifischen 5'-Sequenz der mindestens zweiten FEN-Sonde oder des mindestens einen weiteren Primers (M1) als Markierung vorliegt.

7. Bestätigungsverfahren nach einem der Ansprüche 1 bis 6, wobei
- die Amplifikation der mindestens einen artifiziellen Matrizensequenz mit mindestens einem markierten 5'-Spaltprodukt (S₁₋ₙ) der mindestens einen FEN-Sonde erfolgt und mindestens einem weiteren markierten 5'-Spaltprodukt der mindestens zweiten FEN-Sonde oder mindestens einem markierten weiteren Primer (M1),
- die Markierung der mindestens einen artifiziellen Matrizensequenz mit dem spezifischen Haptenpaar während der Amplifikation erfolgt und
- mindestens ein markierter Doppelstrang des mindestens einen Matrizensequenzamplifikats erhalten wird, welcher am jeweiligen Strang ein Hapten des spezifischen Haptenpaar aufweist.

8. Bestätigungsverfahren nach einem der Ansprüche 1 bis 7, wobei die Bestätigung der mindestens einen nachzuweisenden amplifizierten Zielsequenz das mindestens eine Haptenpaar-markierte artifizielle Matrizensequenzamplifikat ist.

9. Bestätigungsverfahren nach einem der Ansprüche 1 bis 8, wobei das Reaktionsgemisch ferner mindestens ein zur Spaltung der mindestens einen FEN-Sonde geeignetes Enzym umfasst, welches ausgewählt wird aus einer FEN als intrinsischer Bestandteil einer DNA Polymerase oder als ein von einer Polymerase getrennt vorliegendes Enzym.

10. Bestätigungsverfahren nach einem der Ansprüche 1 bis 9, wobei das Reaktionsgemisch ferner mindestens ein FEN-Verstärker-Oligonukleotid (ENH₁₋ₙ) umfasst, dessen Sequenz am 3'-Ende um mindestens eine Base mit der Zielsequenz-spezifischen 3'-Sequenz an der 5'-Bindungsstelle der mindestens einen FEN-Sonde überlappt.

11. Bestätigungsverfahren nach einem der Ansprüche 1 bis 10, wobei das Reaktionsgemisch mindestens zwei artifizielle Matrizensequenzen umfasst, welche sich in der Sequenz und/oder Sequenzlänge unterscheiden und welche jeweils komplementäre Sequenzen zu mindestens einem 5'-Spaltprodukt (S₁₋ₙ) der jeweiligen FEN-Sonde oder zu mindestens einem weiteren Primer (M1) umfassen.

12. Bestätigungsverfahren nach einem der Ansprüche 1 bis 11, wobei das mindestens eine mit einem Haptenpaar-markierte artifizielle Matrizensequenzamplifikat mittels eines immunchromatographischen Verfahrens detektiert wird.

13. Bestätigungsverfahren nach einem der Ansprüche 1 bis 12, wobei das mindestens eine mit einem Haptenpaar-markierte artifizielle Matrizensequenzamplifikat mittels eines Nukleinsäure-Lateral-Flow (NALF) immunchromatographisches Verfahren detektiert wird.

14. Bestätigungsverfahren nach einem der Ansprüche 1 bis 13, wobei das Reaktionsgemisch umfasst
- größer eins bis kleiner gleich 10 unterschiedliche FEN-Sonden, welche entsprechend mit jeweils einem Hapten der größer eins bis kleiner 10 unterschiedlicher spezifischer Haptenpaare markiert sind und
- größer eins bis kleiner gleich 10 unterschiedliche artifizielle Matrizensequenzen, welche jeweils einen komplementären Sequenzabschnitt zu einem markierten 5'-Spaltprodukt (S₁₋ₙ) einer FEN-Sonde umfassen.

15. Bestätigungsverfahren nach einem der Ansprüche 1 bis 14, wobei größer eins bis kleiner gleich 10 unterschiedliche mit jeweils einem spezifischen Haptenpaar-markierte artifizielle Matrizensequenzamplifikate in einem kollektiven und kontinuierlichen Reaktionsgemisch bestätigt und mittels eines immunchromatographischen Verfahrens simultan und distinkt an einer Festphase detektiert werden.

16. Reaktionsgemisch umfassend
- mindestens zwei Zielsequenz-spezifische Primer (P1, P2, P₁₋ₙ), die zur Amplifikation mindestens einer nachzuweisenden Zielsequenz geeignet sind,
- mindestens eine markierte Zielsequenz-spezifische Flap-Endonukleasesonde (FEN-Sonde FEN1, FEN₁₋ₙ), wobei die mindestens eine FEN-Sonde umfasst
• eine Zielsequenz-spezifische 3'-Sequenz, welche komplementär zu einem Sequenzabschnitt der mindestens einen Zielsequenz innerhalb einer Region ist, die von dem mindesten ersten Primer (P1) und dem mindestens zweiten Primer (P2) auf der Zielsequenz begrenzt wird,
• am 3'-Ende der Zielsequenz-spezifischen 3'-Sequenz eine Schutzgruppe und
• eine Zielsequenz-unspezifischen 5'-Sequenz,welche an ihrem 5'-Ende mit einem Hapten eines spezifischen Haptenpaares markiert ist, wobei nach Spaltung der mindestens einen FEN-Sonde, das mindestens eine erhaltene 5'-Spaltprodukt (S₁₋ₙ) mit dem Hapten markiert ist und als Primer zur Amplifikation der mindestens einen artifiziellen Matrizensequenz dient, und
- mindestens eine artifizielle Matrizensequenz, welche komplementäre Sequenzen zu dem mindestens einen 5'-Spaltprodukt (S₁₋ₙ) der mindestens einen FEN-Sonde umfasst.

17. Reaktionsgemisch nach Anspruch 16, welches zusätzlich mindestens einen weiteren Primer (M1) der an den komplementären Strang der mindestens einen artifiziellen Matrizensequenz bindet und/oder mindestens ein FEN-Verstärker-Oligonukleotid (ENH₁₋ₙ) umfasst.

18. Multiplex-Kit umfassend eine Kombination aus
- mindestens einer markierten oder mehr markierten FEN-Sonden, welche sich untereinander unterscheiden, und jede FEN-Sonde jeweils umfasst
• eine Zielsequenz-spezifische 3'-Sequenz, welche komplementär zu einem Sequenzabschnitt mindestens einer Zielsequenz innerhalb einer Region ist, die von mindestens einem ersten Primer (P1) und mindestens einem zweiten Primer (P2) auf der Zielsequenz begrenzt wird,
• am 3'-Ende der Zielsequenz-spezifische 3'-Sequenz eine Schutzgruppe und
• eine Zielsequenz-unspezifischen 5'-Sequenz, welche an ihrem 5'-Ende mit einem Detektor-Hapten oder einem zur Matrizensequenz sequenzspezifisches Hapten eines spezifischen Haptenpaares markiert ist, wobei nach Spaltung der mindestens einen FEN-Sonde das mindestens eine erhaltene 5'-Spaltprodukt (S₁₋ₙ) mit einem Hapten markiert ist und als Primer zur Amplifikation der mindestens einen artifiziellen Matrizensequenz dient, und
- mindestens eine oder mehrere unterschiedliche artifizielle Matrizensequenzen, welche sich in ihrer Sequenz und/oder Sequenzlänge unterscheiden und welche jeweils komplementäre Sequenzen zu mindestens einem 5'-Spaltprodukt (S₁₋ₙ) der mindestens einen FEN-Sonde oder zu mindestens einem weiteren Primer (M1) umfassen.

19. Multiplex-Kit nach Anspruch 18, wobei es mindestens zwei markierte FEN-Sonden (FEN1 und FEN2, FEN₁₋ₙ) und/oder mindestens eine markierte FEN-Sonde und mindestens einen weiteren markierten Primer (M1) umfasst, wobei als Markierung jeweils ein Hapten eines spezifischen Haptenpaares vorliegt.

20. Verwendung eines Reaktionsgemisches nach Anspruch 16 oder 17 und/oder Verwendung eines Multiplex-Kits nach Anspruch 18 oder 19 in einem Verfahren nach einem der Ansprüche 1 bis 15.

## Claims

1. Confirmation of at least one amplified nucleic acid target sequence (target sequence) during a duplication reaction in a collective and continuous reaction setup containing a reaction mixture comprising
- at least one target sequence to be verified,
- at least two target sequence-specific primers (P1, P2, P₁₋ₙ) being suitable for amplification of the at least one target sequence,
- at least one labelled target sequence-specific flap endonuclease probe (FEN probe FEN1, FEN₁₋ₙ), the at least one FEN probe comprising
• a target sequence-specific 3'-sequence which is complementary to a sequence fragment of the at least one target sequence within a region being restricted on the target sequence by the at least first primer (P1) and the at least second primer (P2),
• a protective group at the 3'-end of the target sequence-specific 3'-sequence, and
• a target sequence-unspecific 5'-sequence which is labelled at its 5'-end with one hapten of a specific hapten pair, and
- at least one artificial matrix sequence,
- at least one further primer (M1), the further primer (M1) being complementary to a sequence fragment of the counter strand of the at least one artificial matrix sequence, and/or
- at least one FEN enhancer oligonucleotide (ENH₁₋ₙ)
the confirmation assay comprises per cycle of the duplication reaction the steps of
- hybridisation of the target sequence-specific 3'-sequence of the at least one FEN probe to a complementary sequence of the at least one target sequence to be verified,
- cleavage of the at least one FEN probe,
- obtaining at least one free 5'-cleavage product (S₁₋ₙ) each comprising the target sequence-unspecific 5'-sequence,
- hybridisation of the at least one 5'-cleavage product (S₁₋ₙ) of the at least one FEN probe to a complementary sequence of the at least one artificial matrix sequence,
- amplification of the at least one artificial matrix sequence using the at least one 5'-cleavage product (S₁₋ₙ) of the at least one FEN probe and the at least one primer (M1), and
- optionally, labelling of the at least one artificial matrix sequence during amplification by the at least one 5'-cleavage product (S₁₋ₙ) of the at least one FEN probe.

2. Confirmation assay according to claim 1, wherein the 5'-end of the target sequence-unspecific 5'-sequence of the at least one FEN probe is labelled with a detector hapten or one hapten of a specific hapten pair, being sequence-specific to the matrix sequence.

3. Confirmation assay according to claim 1 or 2, wherein the content of the at least one target sequence to be verified is greater than or equal to 2 copies in the reaction mixture.

4. Confirmation assay according to any one of the claims 1 to 3, wherein the at least one target sequence to be verified is present within a biological sample, preferably a human sample.

5. Confirmation assay according to any one of the claims 1 to 4, wherein the reaction mixture comprises
- at least two labelled FEN probes (FEN1 and FEN2, FEN₁₋ₙ), each comprising a target sequence-specific 3'-sequence.

6. Confirmation assay according to any one of the claims 1 to 5, wherein the other hapten of the specific hapten pair is present as labelling at the 5'-end of the target sequence-unspecific 5'-sequence of the at least second FEN probe or of the at least one further primer (M1).

7. Confirmation assay according to any one of the claims 1 to 6, wherein
- amplification of the at least one artificial matrix sequence is carried out using at least one labelled 5'-cleavage product (S₁₋ₙ) of the at least one FEN probe and at least one further labelled 5'-cleavage product of the at least second FEN probe or at least one further labelled primer (M1),
- labelling of the at least one artificial matrix sequence with the specific hapten pair is carried out during amplification, and
- at least one labelled double strand of the at least one matrix sequence amplificate having one hapten of the specific hapten pair at the respective strand is obtained.

8. Confirmation assay according to any one of the claims 1 to 7, wherein the confirmation of the at least one amplified target sequence to be verified is the at least one hapten pair-labelled artificial matrix sequence amplificate.

9. Confirmation assay according to any one of the claims 1 to 8, wherein the reaction mixture further comprises at least one enzyme being suitable for cleavage of the at least one FEN probe, which is selected from a FEN as intrinsic component of a DNA polymerase or as enzyme being separated from a polymerase.

10. Confirmation assay according to any one of the claims 1 to 9, wherein the reaction mixture further comprises at least one FEN enhancer oligonucleotide (ENH₁₋ₙ), the sequence of which at the 3'-end overlaps by at least one base with the target sequence-specific 3'-sequence at the 5'-binding site of the at least one FEN probe.

11. Confirmation assay according to any one of the claims 1 to 10, wherein the reaction mixture comprises at least two artificial matrix sequences differing in sequence and/or in sequence length and each comprising complementary sequences to at least one 5'-cleavage product (S₁₋ₙ) of the respective FEN probe or to at least one further primer (M1).

12. Confirmation assay according to any one of the claims 1 to 11, wherein the at least one artificial matrix sequence amplificate labelled with a hapten pair is detected by means of an immunochromatographic method.

13. Confirmation assay according to any one of the claims 1 to 12, wherein the at least one artificial matrix sequence amplificate labelled with a hapten pair is detected by means of a nucleic acid lateral flow (NALF) immunochromatographic method.

14. Confirmation assay according to any one of the claims 1 to 13, wherein the reaction mixture comprises
- greater than one to less than or equal to 10 different FEN probes correspondingly being labelled with one hapten each of the greater than one to less than 10 different specific hapten pairs, and
- greater than one to less than or equal to 10 different artificial matrix sequences each comprising a complementary sequence fragment to a labelled 5'-cleavage product (S₁₋ₙ) of a FEN probe.

15. Confirmation assay according to any one of the claims 1 to 14, wherein greater than one to less than or equal to 10 different artificial matrix sequence amplificates labelled with respectively one specific hapten pair are confirmed in a collective and continuous reaction mixture, and are simultaneously and distinctly detected at a solid phase by means of an immunochromatographic method.

16. Reaction mixture comprising
- at least two target sequence-specific primers (P1, P2, P₁₋ₙ) being suitable for amplification of at least one target sequence to be verified,
- at least one labelled target sequence-specific flap endonuclease probe (FEN probe FEN1, FEN₁₋ₙ), the at least one FEN probe comprising
• a target sequence-specific 3'-sequence which is complementary to a sequence fragment of the at least one target sequence within a region being restricted on the target sequence by the at least first primer (P1) and the at least second primer (P2),
• a protective group at the 3'-end of the target sequence-specific 3'-sequence, and
• a target sequence-unspecific 5'-sequence which is labelled at its 5'-end with one hapten of a specific hapten pair, the at least one 5'-cleavage product (S₁₋ₙ) obtained being labelled with the hapten after cleavage of the at least one FEN probe and serving as primer for amplification of the at least one artificial matrix sequence, and
- at least one artificial matrix sequence comprising complementary sequences to the at least one 5'-cleavage product (S₁₋ₙ) of the at least one FEN probe.

17. Reaction mixture according to claim 16, which additionally comprises at least one further primer (M1) binding to the complementary strand of the at least one artificial matrix sequence, and/or at least one FEN enhancer oligonucleotide (ENH₁₋ₙ).

18. Multiplex kit comprising a combination of
- at least one labelled or more labelled FEN probes differing from one another, and each FEN probe respectively comprises
• a target sequence-specific 3'-sequence which is complementary to a sequence fragment of the at least one target sequence within a region being restricted on the target sequence by at least one first primer (P1) and at least one second primer (P2),
• a protective group at the 3'-end of the target sequence-specific 3'-sequence, and
• a target sequence-unspecific 5'-sequence which is labelled at its 5'-end with a detector hapten or one hapten of a specific hapten pair being sequence-specific to the matrix sequence, the at least one 5'-cleavage product (S₁₋ₙ) obtained being labelled with a hapten after cleavage of the at least one FEN probe and serving as primer for amplification of the at least one artificial matrix sequence, and
- at least one or more different artificial matrix sequences differing in their sequence and/or in their sequence length and each comprising complementary sequences to at least one 5' cleavage product (S₁₋ₙ) of the at least one FEN probe or to at least one further primer (M1).

19. Multiplex kit according to claim 18, comprising at least two labelled FEN probes (FEN1 and FEN2, FEN₁₋ₙ) and/or at least one labelled FEN probe and at least one further labelled primer (M1), one hapten of a specific hapten pair being present as labelling respectively.

20. Use of a reaction mixture according to claim 16 or 17 and/or use of a multiplex kit according to claim 18 or 19 in a method according to any one of the claims 1 to 15.

## Revendications

1. Confirmation de l'au moins une séquence cible amplifiée de l'acide nucléique (séquence cible) pendant une réaction de duplication dans une préparation de réaction collective et continue contenant un mélange de réaction comprenant
- au moins une séquence cible à être vérifiée,
- au moins deux amorces, spécifique de séquence cible (P1, P2, P₁₋ₙ) étant appropriées pour l'amplification de l'au moins une séquence cible,
- au moins une sonde d'endonucléase flap marquée, spécifique de séquence cible (sonde FEN FEN1, FEN₁₋ₙ), l'au moins une sonde FEN comprenant
• une séquence 3', spécifique de séquence cible, qui est complémentaire à un fragment de séquence de l'au moins une séquence cible dans une région étant restreinte sur la séquence cible par l'au moins première amorce (P1) et l'au moins deuxième amorce (P2),
• un groupe protecteur à l'extrémité 3' de la séquence 3', spécifique de séquence cible, et
• une sequence 5', non spécifique de séquence cible, qui est marquée à son extrémité 5' avec un haptène d'une paire d'haptène spécifique, et
- au moins une séquence matrice artificielle,
- au moins une autre amorce (M1), l'autre amorce (M1) étant complémentaire à un fragment de séquence du brin contraire de l'au moins une séquence matrice artificielle, et/ou
- au moins un oligonucléotide d'amplificateur FEN (ENH₁₋ₙ) le test de confirmation comprend par cycle de la réaction de duplication les étapes de
- l'hybridation de la séquence 3', spécifique de séquence cible, de l'au moins une sonde FEN à la séquence complémentaire de l'au moins une séquence cible à être vérifiée,
- le clivage de l'au moins une sonde FEN,
- obtenir au moins un produit de clivage 5' libre (S₁₋ₙ) chacun comprenant la séquence 5', non spécifique de séquence cible,
- l'hybridisation de l'au moins un produit de clivage 5' (S₁₋ₙ) de l'au moins une sonde FEN à une séquence complémentaire de l'au moins une séquence matrice artificielle,
- l'amplification de l'au moins une séquence matrice artificielle avec l'au moins un produit de clivage 5' (S₁₋ₙ) de l'au moins une sonde FEN et l'au moins une amorce (M1), et
- facultativement, le marquage de l'au moins une séquence matrice artificielle pendant l'amplification par l'au moins un produit de clivage 5'(S₁₋ₙ) de l'au moins une sonde FEN.

2. Test de confirmation selon la revendication 1, selon lequel l'extrémité 5' de la sequence 5', non spécifique de séquence cible, de l'au moins une sonde FEN est marquée avec un haptène détecteur ou un haptène, spécifique de séquence à la séquence matrice, d'une paire d'haptène.

3. Test de confirmation selon la revendication 1 ou 2, selon lequel la teneur de l'au moins une séquence cible à être vérifiée est supérieure ou égale à 2 copies dans le mélange de réaction.

4. Test de confirmation selon l'une des revendications 1 à 3, selon lequel l'au moins une séquence cible à être vérifiée est présente dans un l'échantillon biologique, de préférence un l'échantillon humain.

5. Test de confirmation selon l'une des revendications 1 à 4, selon lequel le mélange de réaction comprend
- au moins deux sondes FEN marquées (FEN1 et FEN2, FEN₁₋ₙ), chacune comprenant une séquence 3', spécifique de séquence cible.

6. Test de confirmation selon l'une des revendications 1 à 5, selon lequel l'autre haptène de la paire d'haptène spécifique est présent au tant qu'un marquage à l'extrémité 5' de la séquence 5', non spécifique de séquence cible, de l'au moins deuxième sonde FEN ou de l'au moins une autre amorce (M1).

7. Test de confirmation selon l'une des revendications 1 à 6, selon lequel
- l'amplification de l'au moins une sequence matrice artificielle est effectuée utilisant au moins un produit de clivage 5' marqué (S₁₋ₙ) de l'au moins une sonde FEN et au moins un autre produit de clivage 5' marqué de l'au moins deuxième sonde FEN ou au moins une autre amorce marquée (M1),
- le marquage de l'au moins une séquence matrice artificielle avec la paire d'haptène spécifique est effectué pendant l'amplification, et
- au moins un double brin marqué de l'au moins un produit amplification de la séquence matrice, ayant au brin respectif un haptène de la paire d'haptène spécifique, est obtenu.

8. Test de confirmation selon l'une des revendications 1 à 7, dans lequel la confirmation de l'au moins une séquence cible amplifiée à être vérifiée est l'au moins un produit d'amplification, marqué par la paire d'haptène, de la séquence matrice artificielle.

9. Test de confirmation selon l'une des revendications 1 à 8, selon lequel le mélange de réaction comprend en outre au moins une enzyme étant appropriée pour le clivage de l'au moins une sonde FEN, qui est sélectionnée parmi une FEN en tant qu'un composant intrinsèque d'une ADN polymérase ou au tant qu'une enzyme étant séparée d'une polymérase.

10. Test de confirmation selon l'une des revendications 1 à 9, selon lequel le mélange de réaction comprend en outre au moins un oligonucléotide d'amplificateur FEN (ENH₁₋ₙ), dont la séquence à l'extrémité 3' chevauche par au moins une base avec la séquence 3', spécifique de séquence cible, au site de liaison 5' de l'au moins une sonde FEN.

11. Test de confirmation selon l'une des revendications 1 à 10, selon lequel le mélange de réaction comprend au moins deux séquences matrice artificielles différant en séquence et/ou longueur de séquence et chacune comprenant des séquences complémentaires à au moins un produit de clivage 5' (S₁₋ₙ) de la sonde FEN respective ou à au moins une autre amorce (M1).

12. Test de confirmation selon l'une des revendications 1 à 11, selon lequel l'au moins un produit d'amplification, marqué par une paire d'haptène, de la séquence matrice artificielle est détecté au moyen d'un procédé immunochromatographique.

13. Test de confirmation selon l'une des revendications 1 à 12, selon lequel l'au moins un produit d'amplification, marqué par une paire d'haptène, de la sequence matrice est détecté au moyen d'un procédé immunochromatographique de flux latéral de l'acide nucléique (NALF).

14. Test de confirmation selon l'une des revendications 1 à 13, selon lequel le mélange de réaction comprend
- supérieure à une à inférieure ou égale à 10 différentes sondes FEN étant conformément marquées avec respectivement un haptène des supérieures une à inférieures à 10 différentes paires d'haptène spécifiques, et
- supérieure à une à inférieure ou égale à 10 différentes séquences matrice artificielles, chacune comprenant un fragment de séquence complémentaire à un produit de clivage 5' marqué (S₁₋ₙ) d'une sonde FEN.

15. Test de confirmation selon l'une des revendications 1 à 14, selon lequel supérieur à un à inférieur ou égal à 10 différents produits d'amplification, marquée avec respectivement une paire d'haptène spécifique, de la séquence matrice sont confirmés dans un mélange de réaction collectif et continu, est sont détectés simultanément et distinctement à une phase solide au moyen d'un procédé immunochromatographique.

16. Mélange de reaction comprenant
- au moins deux amorces, spécifique de séquence cible (P1, P2, P₁₋ₙ) étant appropriées pour l'amplification de l'au moins une séquence cible,
- au moins une sonde d'endonucléase flap marquée, spécifique de séquence cible (sonde FEN FEN1, FEN₁₋ₙ), l'au moins une sonde FEN comprenant
· une séquence 3', spécifique de séquence cible, qui est complémentaire à un fragment de séquence de l'au moins une séquence cible dans une région étant restreinte sur la séquence cible par l'au moins première amorce (P1) et l'au moins deuxième amorce (P2),
· un groupe protecteur à l'extrémité 3' de la séquence 3', spécifique de séquence cible, et
· une sequence 5', non spécifique de séquence cible, qui est marquée à son extrémité 5' avec un haptène d'une paire d'haptène spécifique, l'au moins un produit de clivage 5' (S₁₋ₙ) obtenu étant marqué avec l'haptène après le clivage de l'au moins une sonde FEN et servant de l'amorce pour l'amplification de l'au moins une séquence matrice artificielle, et
- au moins une séquence matrice artificielle comprenant des séquences complémentaires á l'au moins un produit de clivage 5' (S₁₋ₙ) de l'au moins une sonde FEN.

17. Mélange de réaction selon la revendication 16, qui comprend additionnellement au moins une autre amorce (M1) liant au brin complémentaire d'au moins une séquence matrice artificielle, et/ou au moins un oligonucléotide d'amplificateur FEN (ENH₁₋ₙ).

18. Kit multiplexe comprenant une combinaison de
- au moins une ou plus sondes FEN marquées différant l'une à l'autre, et chaque sonde FEN comprend respectivement
· une séquence 3', spécifique de séquence cible, qui est complémentaire à un fragment de séquence de l'au moins une séquence cible dans une région étant restreinte sur la séquence cible par l'au moins première amorce (P1) et l'au moins deuxième amorce (P2),
· un groupe protecteur à l'extrémité 3' de la séquence 3', spécifique de séquence cible, et
· une sequence 5', non spécifique de séquence cible, qui est marquée à son extrémité 5' avec un haptène détecteur ou un haptène, étant spécifique de séquence à la séquence matrice, d'une paire d'haptène spécifique, l'au moins un produit de clivage 5' (S₁₋ₙ) obtenu étant marqué avec un haptène après le clivage de l'au moins une sonde FEN et servant de l'amorce pour l'amplification de l'au moins une séquence matrice artificielle, et
- au moins une ou plus différentes séquences matrice artificielle différant en leur séquence et/ou en leur longueur de séquence et chacune comprenant des séquences complémentaires á au moins un produit de clivage 5' (S₁₋ₙ) de l'au moins une sonde FEN ou à au moins une autre amorce (M1).

19. Kit multiplexe selon la revendication 18, comprenant au moins deux sondes FEN marquées (FEN1 et FEN2, FEN₁₋ₙ) et/ou au moins une sonde FEN marquée et au moins une autre amorce marquée (M1), un haptène d'une paire d'haptène spécifique étant présent au tant qu'un marquage respectivement.

20. Utilisation d'un mélange de réaction selon la revendication 16 ou 17 et/ou utilisation d'un kit multiplexe selon la revendication 18 ou 19 dans un procédé selon l'une des revendications 1 à 15.
